# EUROPEAN PATENT APPLICATION

(11) **EP 4 534 656 A1**
(43) Date of publication of application: **09.04.2025**
(21) Application number: 23816176.4
(22) Date of filing: 29.05.2023
(51) Int. Cl.: C12N 5/10, C12N 1/15, C12N 1/19, C12N 1/21, C12N 15/54, C12N 15/63, C12Q 1/48, C12Q 1/68, C12N 9/10

(54) **DNA METHYLTRANSFERASE**

(30) Priority: 30.05.2022 JP 2022087994
(71) Applicant: Japan Science and Technology Agency, Kawaguchi-shi, Saitama 332-0012 (JP)
(72) Inventor: MIURA, Fumihito, Fukuoka-shi, Fukuoka 819-0395 (JP)
(74) Representative: Simmons & Simmons LLP (Munich)
(86) International application number: PCT/JP2023/020732
(87) International publication number: WO 2023/234419

(57) **Abstract**

The present invention provides a DNA methyltransferase that is capable of recognizing and methylating a short context other than but equivalent to a CG dinucleotide sequence where endogenous methylation occurs, etc. The present invention relates to a protein modified to delete some amino acid residues including the N-terminal end of DNA methyltransferase M.CviQIX or M.CviPII and thereby acquire the ability to specifically recognize a CC dinucleotide sequence and 5-methylate the 5'-position cytosine residue in the sequence, etc.

SEQUENCE LISTING

<110>  Kyushu University, National University Corporation

<120>  DNA methyltransferase

<130>  G3130WO

<150>  JP 2022-087994
<151>  2022-05-30

<160>  10

<170>  PatentIn version 3.5

<210>  1
<211>  1089
<212>  DNA
<213>  Paramecium bursaria Chlorella virus NY2A

<220>
<221>  CDS
<222>  (1)..(1089)

<400>  1
atg cgc acg aaa tac cgc atc ttc atc gtc tgc atg ctg atg tcc atg      48
Met Arg Thr Lys Tyr Arg Ile Phe Ile Val Cys Met Leu Met Ser Met
1               5                   10                  15

aaa gcc ttg gaa cta ttt gcg ggg ata ggt ggc att acc cat ggc tta      96
Lys Ala Leu Glu Leu Phe Ala Gly Ile Gly Gly Ile Thr His Gly Leu
            20                  25                  30

cgt ggc tat gta gaa ccg att gca ttc tgc gaa tat gag aaa gat gcc      144
Arg Gly Tyr Val Glu Pro Ile Ala Phe Cys Glu Tyr Glu Lys Asp Ala
        35                  40                  45

agc tcg ttt ctg tca caa cgt ggc tta ccc gtt cat ggg gac att acg      192
Ser Ser Phe Leu Ser Gln Arg Gly Leu Pro Val His Gly Asp Ile Thr
    50                  55                  60

aag ttt gac gcc agt gtg tac aaa aac aag att gac att gtg aca gca      240
Lys Phe Asp Ala Ser Val Tyr Lys Asn Lys Ile Asp Ile Val Thr Ala
65                  70                  75                  80

gga tgg cct tgt act ggg ttt tcg acc gcg ggt aaa ggc act ggt ttt      288
Gly Trp Pro Cys Thr Gly Phe Ser Thr Ala Gly Lys Gly Thr Gly Phe
                85                  90                  95

gag cat gaa gct agc ggt tta tgg aca gaa gtg gtc cga gta gtg aaa      336
Glu His Glu Ala Ser Gly Leu Trp Thr Glu Val Val Arg Val Val Lys
            100                 105                 110

gag agc gaa ccg aaa tac gtc ttt ttg gaa aat agt cac gtt ttg gcg 384
Glu Ser Glu Pro Lys Tyr Val Phe Leu Glu Asn Ser His Val Leu Ala
        115                 120                 125

caa acc aaa aac ctc aag gtc atc atc cat gat ctg gat aac ctt ggc 432
Gln Thr Lys Asn Leu Lys Val Ile Ile His Asp Leu Asp Asn Leu Gly
    130                 135                 140

tat gat acg cgt tgg tgg acg tgc aga tcg aat gac gtt aat gtc ggt 480
Tyr Asp Thr Arg Trp Trp Thr Cys Arg Ser Asn Asp Val Asn Val Gly
145                 150                 155                 160

gca cat cac aat cgg tat cgc tgg ttt atg ctc gca gag aaa aaa ggc 528
Ala His His Asn Arg Tyr Arg Trp Phe Met Leu Ala Glu Lys Lys Gly
                165                 170                 175

tct gtg acc aaa ttc gtg aaa att cag gtg aaa aag ttc aat tgg tct 576
Ser Val Thr Lys Phe Val Lys Ile Gln Val Lys Lys Phe Asn Trp Ser
            180                 185                 190

ggt gac ttc aaa gag aaa cag ata agc gag aac agt cac gaa aac aag 624
Gly Asp Phe Lys Glu Lys Gln Ile Ser Glu Asn Ser His Glu Asn Lys
        195                 200                 205

cag ctc att aag ttc atg gga aac tcc gta gtt ccg gat caa gtt cgc 672
Gln Leu Ile Lys Phe Met Gly Asn Ser Val Val Pro Asp Gln Val Arg
    210                 215                 220

tat gcg ttt gaa tcg atg agc gac atg att ctg gaa ggc agt ctg gtg 720
Tyr Ala Phe Glu Ser Met Ser Asp Met Ile Leu Glu Gly Ser Leu Val
225                 230                 235                 240

aat gaa aag gat gag atc gtg aag gta ggc tat tcc aaa gat agc atc 768
Asn Glu Lys Asp Glu Ile Val Lys Val Gly Tyr Ser Lys Asp Ser Ile
                245                 250                 255

atg tac aaa att ccg att gaa cac aaa atc att ccg aaa ctt aac atc 816
Met Tyr Lys Ile Pro Ile Glu His Lys Ile Ile Pro Lys Leu Asn Ile
            260                 265                 270

gtt ctg acc cca cgt gat ccg cca gaa ggc cat aaa gcc cgt gaa gaa 864
Val Leu Thr Pro Arg Asp Pro Pro Glu Gly His Lys Ala Arg Glu Glu
        275                 280                 285

gcg atc atc aaa agc ccc att ctg atg acg tac tgg aat act ccg gcc 912
Ala Ile Ile Lys Ser Pro Ile Leu Met Thr Tyr Trp Asn Thr Pro Ala
    290                 295                 300

ttt tgc tat cac aaa tca gct cgc ggc gct aaa att ctg acc aaa cgt 960
Phe Cys Tyr His Lys Ser Ala Arg Gly Ala Lys Ile Leu Thr Lys Arg
305                 310                 315                 320

cag aaa aac aac ctg cat acc cag atc aaa ttc tgt cct ggt ggt tct 1008
Gln Lys Asn Asn Leu His Thr Gln Ile Lys Phe Cys Pro Gly Gly Ser

325                         330                         335

gac gat ggt tat ctg tca ggt cgc ttt tgt gcg tgg ctg atg gga tac         1056
Asp Asp Gly Tyr Leu Ser Gly Arg Phe Cys Ala Trp Leu Met Gly Tyr
            340                 345                 350

gat gat gaa tac ctg ggg gat ctt atg cac tat                             1089
Asp Asp Glu Tyr Leu Gly Asp Leu Met His Tyr
        355                 360

<210>  2
<211>  363
<212>  PRT
<213>  Paramecium bursaria Chlorella virus NY2A

<400>  2

Met Arg Thr Lys Tyr Arg Ile Phe Ile Val Cys Met Leu Met Ser Met
1               5                   10                  15

Lys Ala Leu Glu Leu Phe Ala Gly Ile Gly Gly Ile Thr His Gly Leu
            20                  25                  30

Arg Gly Tyr Val Glu Pro Ile Ala Phe Cys Glu Tyr Glu Lys Asp Ala
        35                  40                  45

Ser Ser Phe Leu Ser Gln Arg Gly Leu Pro Val His Gly Asp Ile Thr
    50                  55                  60

Lys Phe Asp Ala Ser Val Tyr Lys Asn Lys Ile Asp Ile Val Thr Ala
65                  70                  75                  80

Gly Trp Pro Cys Thr Gly Phe Ser Thr Ala Gly Lys Gly Thr Gly Phe
                85                  90                  95

Glu His Glu Ala Ser Gly Leu Trp Thr Glu Val Val Arg Val Val Lys
            100                 105                 110

Glu Ser Glu Pro Lys Tyr Val Phe Leu Glu Asn Ser His Val Leu Ala
        115                 120                 125

Gln Thr Lys Asn Leu Lys Val Ile Ile His Asp Leu Asp Asn Leu Gly
    130                 135                 140

Tyr Asp Thr Arg Trp Trp Thr Cys Arg Ser Asn Asp Val Asn Val Gly
145                 150                 155                 160

Ala His His Asn Arg Tyr Arg Trp Phe Met Leu Ala Glu Lys Lys Gly
                165                 170                 175

Ser Val Thr Lys Phe Val Lys Ile Gln Val Lys Lys Phe Asn Trp Ser
            180                 185                 190

Gly Asp Phe Lys Glu Lys Gln Ile Ser Glu Asn Ser His Glu Asn Lys
        195                 200                 205

Gln Leu Ile Lys Phe Met Gly Asn Ser Val Val Pro Asp Gln Val Arg
    210                 215                 220

Tyr Ala Phe Glu Ser Met Ser Asp Met Ile Leu Glu Gly Ser Leu Val
225                 230                 235                 240

Asn Glu Lys Asp Glu Ile Val Lys Val Gly Tyr Ser Lys Asp Ser Ile
                245                 250                 255

Met Tyr Lys Ile Pro Ile Glu His Lys Ile Ile Pro Lys Leu Asn Ile
            260                 265                 270

Val Leu Thr Pro Arg Asp Pro Pro Glu Gly His Lys Ala Arg Glu Glu
        275                 280                 285

Ala Ile Ile Lys Ser Pro Ile Leu Met Thr Tyr Trp Asn Thr Pro Ala
    290                 295                 300

Phe Cys Tyr His Lys Ser Ala Arg Gly Ala Lys Ile Leu Thr Lys Arg
305                 310                 315                 320

Gln Lys Asn Asn Leu His Thr Gln Ile Lys Phe Cys Pro Gly Gly Ser
                325                 330                 335

Asp Asp Gly Tyr Leu Ser Gly Arg Phe Cys Ala Trp Leu Met Gly Tyr
            340                 345                 350

Asp Asp Glu Tyr Leu Gly Asp Leu Met His Tyr
        355                 360

<210> 3
<211> 1089
<212> DNA
<213> Chlorella virus

<220>
<221> CDS
<222> (1)..(1089)

<400> 3
atg cgc acg aaa tac cgc atc ttc atc gtc tgc atg ctg atg tcc atg 48
Met Arg Thr Lys Tyr Arg Ile Phe Ile Val Cys Met Leu Met Ser Met
1               5                   10                  15

aaa gcc ttg gaa cta ttt gcg ggg ata ggt ggc att acc cat ggc tta 96
Lys Ala Leu Glu Leu Phe Ala Gly Ile Gly Gly Ile Thr His Gly Leu
            20                  25                  30

cgt ggc tat gta gaa ccg att gca ttc tgc gaa tat gag aaa gat gcc 144
Arg Gly Tyr Val Glu Pro Ile Ala Phe Cys Glu Tyr Glu Lys Asp Ala
        35                  40                  45

agc tcg ttt ctg tca caa cgt ggc tta ccc gtt cat ggg gac att acg 192
Ser Ser Phe Leu Ser Gln Arg Gly Leu Pro Val His Gly Asp Ile Thr
    50                  55                  60

aag ttt gac gcc agt gtg tac aaa aac aag att gac att gtg aca gca 240
Lys Phe Asp Ala Ser Val Tyr Lys Asn Lys Ile Asp Ile Val Thr Ala
65                  70                  75                  80

gga tgg cct tgt act ggg ttt tcg acc gcg ggt aaa ggc act ggt ttt 288
Gly Trp Pro Cys Thr Gly Phe Ser Thr Ala Gly Lys Gly Thr Gly Phe
                85                  90                  95

gag cat gaa gct agc ggt tta tgg aca gaa gtg gtc cga gta gtg aaa 336
Glu His Glu Ala Ser Gly Leu Trp Thr Glu Val Val Arg Val Val Lys
            100                 105                 110

gag agc gaa ccg aaa tac gtc ttt ttg gaa aat agt cac gtt ttg gcg 384
Glu Ser Glu Pro Lys Tyr Val Phe Leu Glu Asn Ser His Val Leu Ala
        115                 120                 125

caa acc aaa aac ctc aag gtc atc atc cat gat ctg gat atc ctt ggc 432
Gln Thr Lys Asn Leu Lys Val Ile Ile His Asp Leu Asp Ile Leu Gly
    130                 135                 140

tat gat acg cgt tgg tgg acg tgc aga tcg aat gac gtt aat gtc ggt 480
Tyr Asp Thr Arg Trp Trp Thr Cys Arg Ser Asn Asp Val Asn Val Gly
145                 150                 155                 160

gca cat cac aat cgg tat cgc tgg ttt atg ctc gca gag aaa aaa ggc 528

Ala His His Asn Arg Tyr Arg Trp Phe Met Leu Ala Glu Lys Lys Gly
                165                 170                 175

tct gtg acc aaa ttc gtg aaa att cag gtg aaa aag ttc aat tgg tct      576
Ser Val Thr Lys Phe Val Lys Ile Gln Val Lys Lys Phe Asn Trp Ser
            180                 185                 190

ggt gac ttc aaa gag aaa cag ata agc gag aac agt cac gaa aac aag      624
Gly Asp Phe Lys Glu Lys Gln Ile Ser Glu Asn Ser His Glu Asn Lys
        195                 200                 205

cag ctc att aag ttc atg gga aac tcc gta gtt ccg gat caa gtt cgc      672
Gln Leu Ile Lys Phe Met Gly Asn Ser Val Val Pro Asp Gln Val Arg
    210                 215                 220

tat gcg ttt gaa tcg atg agc gac atg att ctg gaa ggc agt ctg gtg      720
Tyr Ala Phe Glu Ser Met Ser Asp Met Ile Leu Glu Gly Ser Leu Val
225                 230                 235                 240

aat gat aag gat gag atc gtg aag gta ggc tat tcc aaa gat ggc atc      768
Asn Asp Lys Asp Glu Ile Val Lys Val Gly Tyr Ser Lys Asp Gly Ile
                245                 250                 255

atg tac aaa att ccg att gaa cac aaa atc att ccg aaa ctt aac atc      816
Met Tyr Lys Ile Pro Ile Glu His Lys Ile Ile Pro Lys Leu Asn Ile
            260                 265                 270

gtt ctg acc cca cgt gat ccg cca gaa ggc cat aaa gcc cgt gaa gaa      864
Val Leu Thr Pro Arg Asp Pro Pro Glu Gly His Lys Ala Arg Glu Glu
        275                 280                 285

gcg atc atc aaa agc ccc att ctg atg acg tac tgg aat act ccg gcc      912
Ala Ile Ile Lys Ser Pro Ile Leu Met Thr Tyr Trp Asn Thr Pro Ala
    290                 295                 300

ttt tgc tat cac aaa tca gct cgc ggc gct aaa att ctg acc aaa cgt      960
Phe Cys Tyr His Lys Ser Ala Arg Gly Ala Lys Ile Leu Thr Lys Arg
305                 310                 315                 320

cag aaa aac aac ctg cat acc cag atc aaa ttc tgt cct ggt ggt tct     1008
Gln Lys Asn Asn Leu His Thr Gln Ile Lys Phe Cys Pro Gly Gly Ser
                325                 330                 335

gac gat ggt tat ctg tca ggt cgc ttt tgt gcg tgg ctg atg gga tac     1056
Asp Asp Gly Tyr Leu Ser Gly Arg Phe Cys Ala Trp Leu Met Gly Tyr
            340                 345                 350

gat gat gaa tac ctg ggg gat ctt atg cac tat                         1089
Asp Asp Glu Tyr Leu Gly Asp Leu Met His Tyr
        355                 360

<210>  4
<211>  363

<212>  PRT
<213>  Chlorella virus

<400>  4

Met Arg Thr Lys Tyr Arg Ile Phe Ile Val Cys Met Leu Met Ser Met
1               5                   10                  15

Lys Ala Leu Glu Leu Phe Ala Gly Ile Gly Gly Ile Thr His Gly Leu
            20                  25                  30

Arg Gly Tyr Val Glu Pro Ile Ala Phe Cys Glu Tyr Glu Lys Asp Ala
        35                  40                  45

Ser Ser Phe Leu Ser Gln Arg Gly Leu Pro Val His Gly Asp Ile Thr
    50                  55                  60

Lys Phe Asp Ala Ser Val Tyr Lys Asn Lys Ile Asp Ile Val Thr Ala
65                  70                  75                  80

Gly Trp Pro Cys Thr Gly Phe Ser Thr Ala Gly Lys Gly Thr Gly Phe
                85                  90                  95

Glu His Glu Ala Ser Gly Leu Trp Thr Glu Val Val Arg Val Val Lys
            100                 105                 110

Glu Ser Glu Pro Lys Tyr Val Phe Leu Glu Asn Ser His Val Leu Ala
        115                 120                 125

Gln Thr Lys Asn Leu Lys Val Ile Ile His Asp Leu Asp Ile Leu Gly
    130                 135                 140

Tyr Asp Thr Arg Trp Trp Thr Cys Arg Ser Asn Asp Val Asn Val Gly
145                 150                 155                 160

Ala His His Asn Arg Tyr Arg Trp Phe Met Leu Ala Glu Lys Lys Gly
                165                 170                 175

Ser Val Thr Lys Phe Val Lys Ile Gln Val Lys Lys Phe Asn Trp Ser
            180                 185                 190

Gly Asp Phe Lys Glu Lys Gln Ile Ser Glu Asn Ser His Glu Asn Lys

195                 200                 205

Gln Leu Ile Lys Phe Met Gly Asn Ser Val Val Pro Asp Gln Val Arg
    210                 215                 220

Tyr Ala Phe Glu Ser Met Ser Asp Met Ile Leu Glu Gly Ser Leu Val
225                 230                 235                 240

Asn Asp Lys Asp Glu Ile Val Lys Val Gly Tyr Ser Lys Asp Gly Ile
                245                 250                 255

Met Tyr Lys Ile Pro Ile Glu His Lys Ile Ile Pro Lys Leu Asn Ile
            260                 265                 270

Val Leu Thr Pro Arg Asp Pro Pro Glu Gly His Lys Ala Arg Glu Glu
        275                 280                 285

Ala Ile Ile Lys Ser Pro Ile Leu Met Thr Tyr Trp Asn Thr Pro Ala
    290                 295                 300

Phe Cys Tyr His Lys Ser Ala Arg Gly Ala Lys Ile Leu Thr Lys Arg
305                 310                 315                 320

Gln Lys Asn Asn Leu His Thr Gln Ile Lys Phe Cys Pro Gly Gly Ser
                325                 330                 335

Asp Asp Gly Tyr Leu Ser Gly Arg Phe Cys Ala Trp Leu Met Gly Tyr
            340                 345                 350

Asp Asp Glu Tyr Leu Gly Asp Leu Met His Tyr
        355                 360

<210>  5
<211>  1044
<212>  DNA
<213>  Artificial

<220>
<223>  Recombinant DNA

<220>
<221>  CDS

<222>  (1)..(1044)

<400>  5
atg aaa gcc ttg gaa cta ttt gcg ggg ata ggt ggc att acc cat ggc      48
Met Lys Ala Leu Glu Leu Phe Ala Gly Ile Gly Gly Ile Thr His Gly
1               5                   10                  15

tta cgt ggc tat gta gaa ccg att gca ttc tgc gaa tat gag aaa gat      96
Leu Arg Gly Tyr Val Glu Pro Ile Ala Phe Cys Glu Tyr Glu Lys Asp
            20                  25                  30

gcc agc tcg ttt ctg tca caa cgt ggc tta ccc gtt cat ggg gac att      144
Ala Ser Ser Phe Leu Ser Gln Arg Gly Leu Pro Val His Gly Asp Ile
        35                  40                  45

acg aag ttt gac gcc agt gtg tac aaa aac aag att gac att gtg aca      192
Thr Lys Phe Asp Ala Ser Val Tyr Lys Asn Lys Ile Asp Ile Val Thr
    50                  55                  60

gca gga tgg cct tgt act ggg ttt tcg acc gcg ggt aaa ggc act ggt      240
Ala Gly Trp Pro Cys Thr Gly Phe Ser Thr Ala Gly Lys Gly Thr Gly
65                  70                  75                  80

ttt gag cat gaa gct agc ggt tta tgg aca gaa gtg gtc cga gta gtg      288
Phe Glu His Glu Ala Ser Gly Leu Trp Thr Glu Val Val Arg Val Val
                85                  90                  95

aaa gag agc gaa ccg aaa tac gtc ttt ttg gaa aat agt cac gtt ttg      336
Lys Glu Ser Glu Pro Lys Tyr Val Phe Leu Glu Asn Ser His Val Leu
            100                 105                 110

gcg caa acc aaa aac ctc aag gtc atc atc cat gat ctg gat aac ctt      384
Ala Gln Thr Lys Asn Leu Lys Val Ile Ile His Asp Leu Asp Asn Leu
        115                 120                 125

ggc tat gat acg cgt tgg tgg acg tgc aga tcg aat gac gtt aat gtc      432
Gly Tyr Asp Thr Arg Trp Trp Thr Cys Arg Ser Asn Asp Val Asn Val
    130                 135                 140

ggt gca cat cac aat cgg tat cgc tgg ttt atg ctc gca gag aaa aaa      480
Gly Ala His His Asn Arg Tyr Arg Trp Phe Met Leu Ala Glu Lys Lys
145                 150                 155                 160

ggc tct gtg acc aaa ttc gtg aaa att cag gtg aaa aag ttc aat tgg      528
Gly Ser Val Thr Lys Phe Val Lys Ile Gln Val Lys Lys Phe Asn Trp
                165                 170                 175

tct ggt gac ttc aaa gag aaa cag ata agc gag aac agt cac gaa aac      576
Ser Gly Asp Phe Lys Glu Lys Gln Ile Ser Glu Asn Ser His Glu Asn
            180                 185                 190

aag cag ctc att aag ttc atg gga aac tcc gta gtt ccg gat caa gtt      624
Lys Gln Leu Ile Lys Phe Met Gly Asn Ser Val Val Pro Asp Gln Val
        195                 200                 205

cgc tat gcg ttt gaa tcg atg agc gac atg att ctg gaa ggc agt ctg 672
Arg Tyr Ala Phe Glu Ser Met Ser Asp Met Ile Leu Glu Gly Ser Leu
    210                 215                 220

gtg aat gaa aag gat gag atc gtg aag gta ggc tat tcc aaa gat agc 720
Val Asn Glu Lys Asp Glu Ile Val Lys Val Gly Tyr Ser Lys Asp Ser
225                 230                 235                 240

atc atg tac aaa att ccg att gaa cac aaa atc att ccg aaa ctt aac 768
Ile Met Tyr Lys Ile Pro Ile Glu His Lys Ile Ile Pro Lys Leu Asn
                245                 250                 255

atc gtt ctg acc cca cgt gat ccg cca gaa ggc cat aaa gcc cgt gaa 816
Ile Val Leu Thr Pro Arg Asp Pro Pro Glu Gly His Lys Ala Arg Glu
            260                 265                 270

gaa gcg atc atc aaa agc ccc att ctg atg acg tac tgg aat act ccg 864
Glu Ala Ile Ile Lys Ser Pro Ile Leu Met Thr Tyr Trp Asn Thr Pro
        275                 280                 285

gcc ttt tgc tat cac aaa tca gct cgc ggc gct aaa att ctg acc aaa 912
Ala Phe Cys Tyr His Lys Ser Ala Arg Gly Ala Lys Ile Leu Thr Lys
    290                 295                 300

cgt cag aaa aac aac ctg cat acc cag atc aaa ttc tgt cct ggt ggt 960
Arg Gln Lys Asn Asn Leu His Thr Gln Ile Lys Phe Cys Pro Gly Gly
305                 310                 315                 320

tct gac gat ggt tat ctg tca ggt cgc ttt tgt gcg tgg ctg atg gga 1008
Ser Asp Asp Gly Tyr Leu Ser Gly Arg Phe Cys Ala Trp Leu Met Gly
                325                 330                 335

tac gat gat gaa tac ctg ggg gat ctt atg cac tat 1044
Tyr Asp Asp Glu Tyr Leu Gly Asp Leu Met His Tyr
            340                 345

<210> 6
<211> 348
<212> PRT
<213> Artificial

<220>
<223> Recombinant Protein

<400> 6

Met Lys Ala Leu Glu Leu Phe Ala Gly Ile Gly Gly Ile Thr His Gly
1               5                   10                  15

Leu Arg Gly Tyr Val Glu Pro Ile Ala Phe Cys Glu Tyr Glu Lys Asp
            20                  25                  30

Ala Ser Ser Phe Leu Ser Gln Arg Gly Leu Pro Val His Gly Asp Ile
        35                  40                  45

Thr Lys Phe Asp Ala Ser Val Tyr Lys Asn Lys Ile Asp Ile Val Thr
    50                  55                  60

Ala Gly Trp Pro Cys Thr Gly Phe Ser Thr Ala Gly Lys Gly Thr Gly
65                  70                  75                  80

Phe Glu His Glu Ala Ser Gly Leu Trp Thr Glu Val Val Arg Val Val
                85                  90                  95

Lys Glu Ser Glu Pro Lys Tyr Val Phe Leu Glu Asn Ser His Val Leu
            100                 105                 110

Ala Gln Thr Lys Asn Leu Lys Val Ile Ile His Asp Leu Asp Asn Leu
        115                 120                 125

Gly Tyr Asp Thr Arg Trp Trp Thr Cys Arg Ser Asn Asp Val Asn Val
    130                 135                 140

Gly Ala His His Asn Arg Tyr Arg Trp Phe Met Leu Ala Glu Lys Lys
145                 150                 155                 160

Gly Ser Val Thr Lys Phe Val Lys Ile Gln Val Lys Lys Phe Asn Trp
                165                 170                 175

Ser Gly Asp Phe Lys Glu Lys Gln Ile Ser Glu Asn Ser His Glu Asn
            180                 185                 190

Lys Gln Leu Ile Lys Phe Met Gly Asn Ser Val Val Pro Asp Gln Val
        195                 200                 205

Arg Tyr Ala Phe Glu Ser Met Ser Asp Met Ile Leu Glu Gly Ser Leu
    210                 215                 220

Val Asn Glu Lys Asp Glu Ile Val Lys Val Gly Tyr Ser Lys Asp Ser
225                 230                 235                 240

Ile Met Tyr Lys Ile Pro Ile Glu His Lys Ile Ile Pro Lys Leu Asn
                245                 250                 255

Ile Val Leu Thr Pro Arg Asp Pro Pro Glu Gly His Lys Ala Arg Glu
            260                 265                 270

Glu Ala Ile Ile Lys Ser Pro Ile Leu Met Thr Tyr Trp Asn Thr Pro
        275                 280                 285

Ala Phe Cys Tyr His Lys Ser Ala Arg Gly Ala Lys Ile Leu Thr Lys
    290                 295                 300

Arg Gln Lys Asn Asn Leu His Thr Gln Ile Lys Phe Cys Pro Gly Gly
305                 310                 315                 320

Ser Asp Asp Gly Tyr Leu Ser Gly Arg Phe Cys Ala Trp Leu Met Gly
                325                 330                 335

Tyr Asp Asp Glu Tyr Leu Gly Asp Leu Met His Tyr
            340                 345

<210>  7
<211>  1044
<212>  DNA
<213>  Artificial

<220>
<223>  Recombinant DNA

<220>
<221>  CDS
<222>  (1)..(1044)

<400>  7
atg aaa gcc ttg gaa cta ttt gcg ggg ata ggt ggc att acc cat ggc      48
Met Lys Ala Leu Glu Leu Phe Ala Gly Ile Gly Gly Ile Thr His Gly
1               5                   10                  15

tta cgt ggc tat gta gaa ccg att gca ttc tgc gaa tat gag aaa gat      96
Leu Arg Gly Tyr Val Glu Pro Ile Ala Phe Cys Glu Tyr Glu Lys Asp
            20                  25                  30

gcc agc tcg ttt ctg tca caa cgt ggc tta ccc gtt cat ggg gac att      144
Ala Ser Ser Phe Leu Ser Gln Arg Gly Leu Pro Val His Gly Asp Ile
        35                  40                  45

acg aag ttt gac gcc agt gtg tac aaa aac aag att gac att gtg aca 192
Thr Lys Phe Asp Ala Ser Val Tyr Lys Asn Lys Ile Asp Ile Val Thr
    50                  55                  60

gca gga tgg cct tgt act ggg ttt tcg acc gcg ggt aaa ggc act ggt 240
Ala Gly Trp Pro Cys Thr Gly Phe Ser Thr Ala Gly Lys Gly Thr Gly
65                  70                  75                  80

ttt gag cat gaa gct agc ggt tta tgg aca gaa gtg gtc cga gta gtg 288
Phe Glu His Glu Ala Ser Gly Leu Trp Thr Glu Val Val Arg Val Val
                85                  90                  95

aaa gag agc gaa ccg aaa tac gtc ttt ttg gaa aat agt cac gtt ttg 336
Lys Glu Ser Glu Pro Lys Tyr Val Phe Leu Glu Asn Ser His Val Leu
            100                 105                 110

gcg caa acc aaa aac ctc aag gtc atc atc cat gat ctg gat atc ctt 384
Ala Gln Thr Lys Asn Leu Lys Val Ile Ile His Asp Leu Asp Ile Leu
        115                 120                 125

ggc tat gat acg cgt tgg tgg acg tgc aga tcg aat gac gtt aat gtc 432
Gly Tyr Asp Thr Arg Trp Trp Thr Cys Arg Ser Asn Asp Val Asn Val
    130                 135                 140

ggt gca cat cac aat cgg tat cgc tgg ttt atg ctc gca gag aaa aaa 480
Gly Ala His His Asn Arg Tyr Arg Trp Phe Met Leu Ala Glu Lys Lys
145                 150                 155                 160

ggc tct gtg acc aaa ttc gtg aaa att cag gtg aaa aag ttc aat tgg 528
Gly Ser Val Thr Lys Phe Val Lys Ile Gln Val Lys Lys Phe Asn Trp
                165                 170                 175

tct ggt gac ttc aaa gag aaa cag ata agc gag aac agt cac gaa aac 576
Ser Gly Asp Phe Lys Glu Lys Gln Ile Ser Glu Asn Ser His Glu Asn
            180                 185                 190

aag cag ctc att aag ttc atg gga aac tcc gta gtt ccg gat caa gtt 624
Lys Gln Leu Ile Lys Phe Met Gly Asn Ser Val Val Pro Asp Gln Val
        195                 200                 205

cgc tat gcg ttt gaa tcg atg agc gac atg att ctg gaa ggc agt ctg 672
Arg Tyr Ala Phe Glu Ser Met Ser Asp Met Ile Leu Glu Gly Ser Leu
    210                 215                 220

gtg aat gat aag gat gag atc gtg aag gta ggc tat tcc aaa gat ggc 720
Val Asn Asp Lys Asp Glu Ile Val Lys Val Gly Tyr Ser Lys Asp Gly
225                 230                 235                 240

atc atg tac aaa att ccg att gaa cac aaa atc att ccg aaa ctt aac 768
Ile Met Tyr Lys Ile Pro Ile Glu His Lys Ile Ile Pro Lys Leu Asn
                245                 250                 255

atc gtt ctg acc cca cgt gat ccg cca gaa ggc cat aaa gcc cgt gaa 816
Ile Val Leu Thr Pro Arg Asp Pro Pro Glu Gly His Lys Ala Arg Glu

260                 265                 270

gaa gcg atc atc aaa agc ccc att ctg atg acg tac tgg aat act ccg        864
Glu Ala Ile Ile Lys Ser Pro Ile Leu Met Thr Tyr Trp Asn Thr Pro
        275                 280                 285

gcc ttt tgc tat cac aaa tca gct cgc ggc gct aaa att ctg acc aaa        912
Ala Phe Cys Tyr His Lys Ser Ala Arg Gly Ala Lys Ile Leu Thr Lys
    290                 295                 300

cgt cag aaa aac aac ctg cat acc cag atc aaa ttc tgt cct ggt ggt        960
Arg Gln Lys Asn Asn Leu His Thr Gln Ile Lys Phe Cys Pro Gly Gly
305                 310                 315                 320

tct gac gat ggt tat ctg tca ggt cgc ttt tgt gcg tgg ctg atg gga       1008
Ser Asp Asp Gly Tyr Leu Ser Gly Arg Phe Cys Ala Trp Leu Met Gly
                325                 330                 335

tac gat gat gaa tac ctg ggg gat ctt atg cac tat                       1044
Tyr Asp Asp Glu Tyr Leu Gly Asp Leu Met His Tyr
            340                 345

<210>  8
<211>  348
<212>  PRT
<213>  Artificial

<220>
<223>  Recombinant Protein

<400>  8

Met Lys Ala Leu Glu Leu Phe Ala Gly Ile Gly Gly Ile Thr His Gly
1               5                   10                  15

Leu Arg Gly Tyr Val Glu Pro Ile Ala Phe Cys Glu Tyr Glu Lys Asp
            20                  25                  30

Ala Ser Ser Phe Leu Ser Gln Arg Gly Leu Pro Val His Gly Asp Ile
        35                  40                  45

Thr Lys Phe Asp Ala Ser Val Tyr Lys Asn Lys Ile Asp Ile Val Thr
    50                  55                  60

Ala Gly Trp Pro Cys Thr Gly Phe Ser Thr Ala Gly Lys Gly Thr Gly
65                  70                  75                  80

Phe Glu His Glu Ala Ser Gly Leu Trp Thr Glu Val Val Arg Val Val

                85                  90                  95

Lys Glu Ser Glu Pro Lys Tyr Val Phe Leu Glu Asn Ser His Val Leu
            100                 105                 110

Ala Gln Thr Lys Asn Leu Lys Val Ile Ile His Asp Leu Asp Ile Leu
        115                 120                 125

Gly Tyr Asp Thr Arg Trp Trp Thr Cys Arg Ser Asn Asp Val Asn Val
    130                 135                 140

Gly Ala His His Asn Arg Tyr Arg Trp Phe Met Leu Ala Glu Lys Lys
145                 150                 155                 160

Gly Ser Val Thr Lys Phe Val Lys Ile Gln Val Lys Lys Phe Asn Trp
                165                 170                 175

Ser Gly Asp Phe Lys Glu Lys Gln Ile Ser Glu Asn Ser His Glu Asn
            180                 185                 190

Lys Gln Leu Ile Lys Phe Met Gly Asn Ser Val Val Pro Asp Gln Val
        195                 200                 205

Arg Tyr Ala Phe Glu Ser Met Ser Asp Met Ile Leu Glu Gly Ser Leu
    210                 215                 220

Val Asn Asp Lys Asp Glu Ile Val Lys Val Gly Tyr Ser Lys Asp Gly
225                 230                 235                 240

Ile Met Tyr Lys Ile Pro Ile Glu His Lys Ile Ile Pro Lys Leu Asn
                245                 250                 255

Ile Val Leu Thr Pro Arg Asp Pro Pro Glu Gly His Lys Ala Arg Glu
            260                 265                 270

Glu Ala Ile Ile Lys Ser Pro Ile Leu Met Thr Tyr Trp Asn Thr Pro
        275                 280                 285

Ala Phe Cys Tyr His Lys Ser Ala Arg Gly Ala Lys Ile Leu Thr Lys
    290                 295                 300

Arg Gln Lys Asn Asn Leu His Thr Gln Ile Lys Phe Cys Pro Gly Gly
305                 310                 315                 320

Ser Asp Asp Gly Tyr Leu Ser Gly Arg Phe Cys Ala Trp Leu Met Gly
                325                 330                 335

Tyr Asp Asp Glu Tyr Leu Gly Asp Leu Met His Tyr
            340                 345

<210>  9
<211>  1101
<212>  DNA
<213>  Artificial

<220>
<223>  Synthetic DNA

<400>  9
ggatccatgc gcacgaaata ccgcatcttc atcgtctgca tgctgatgtc catgaaagcc      60

ttggaactat ttgcggggat aggtggcatt acccatggct tacgtggcta tgtagaaccg     120

attgcattct gcgaatatga gaaagatgcc agctcgtttc tgtcacaacg tggcttaccc     180

gttcatgggg acattacgaa gtttgacgcc agtgtgtaca aaaacaagat tgacattgtg     240

acagcaggat ggccttgtac tgggttttcg accgcgggta aaggcactgg ttttgagcat     300

gaagctagcg gtttatggac agaagtggtc cgagtagtga aagagagcga accgaaatac     360

gtctttttgg aaaatagtca cgttttggcg caaaccaaaa acctcaaggt catcatccat     420

gatctggata accttggcta tgatacgcgt tggtggacgt gcagatcgaa tgacgttaat     480

gtcggtgcac atcacaatcg gtatcgctgg tttatgctcg cagagaaaaa aggctctgtg     540

accaaattcg tgaaaattca ggtgaaaaag ttcaattggt ctggtgactt caaagagaaa     600

cagataagcg agaacagtca cgaaaacaag cagctcatta agttcatggg aaactccgta     660

gttccggatc aagttcgcta tgcgtttgaa tcgatgagcg acatgattct ggaaggcagt     720

ctggtgaatg aaaaggatga gatcgtgaag gtaggctatt ccaaagatag catcatgtac     780

aaaattccga ttgaacacaa aatcattccg aaacttaaca tcgttctgac cccacgtgat      840

ccgccagaag gccataaagc ccgtgaagaa gcgatcatca aaagccccat tctgatgacg     900

tactggaata ctccggcctt ttgctatcac aaatcagctc gcggcgctaa aattctgacc     960

aaacgtcaga aaaacaacct gcatacccag atcaaattct gtcctggtgg ttctgacgat 1020

ggttatctgt caggtcgctt ttgtgcgtgg ctgatgggat acgatgatga atacctgggg 1080

gatcttatgc actatgaatt c 1101

<210> 10
<211> 1110
<212> DNA
<213> Artificial

<220>
<223> Synthetic DNA

<400> 10
atgaaagccg gatccatgcg cacgaaatac cgcatcttca tcgtctgcat gctgatgtcc 60

atgaaagcct tggaactatt tgcggggata ggtggcatta cccatggctt acgtggctat 120

gtagaaccga ttgcattctg cgaatatgag aaagatgcca gctcgtttct gtcacaacgt 180

ggcttacccg ttcatgggga cattacgaag tttgacgcca gtgtgtacaa aaacaagatt 240

gacattgtga cagcaggatg gccttgtact gggttttcga ccgcgggtaa aggcactggt 300

tttgagcatg aagctagcgg tttatggaca gaagtggtcc gagtagtgaa agagagcgaa 360

ccgaaatacg tctttttgga aaatagtcac gttttggcgc aaaccaaaaa cctcaaggtc 420

atcatccatg atctggatat ccttggctat gatacgcgtt ggtggacgtg cagatcgaat 480

gacgttaatg tcggtgcaca tcacaatcgg tatcgctggt ttatgctcgc agagaaaaaa 540

ggctctgtga ccaaattcgt gaaaattcag gtgaaaaagt tcaattggtc tggtgacttc 600

aaagagaaac agataagcga gaacagtcac gaaaacaagc agctcattaa gttcatggga 660

aactccgtag ttccggatca agttcgctat gcgtttgaat cgatgagcga catgattctg 720

gaaggcagtc tggtgaatga taaggatgag atcgtgaagg taggctattc caaagatggc 780

atcatgtaca aaattccgat tgaacacaaa atcattccga aacttaacat cgttctgacc 840

ccacgtgatc cgccagaagg ccataaagcc cgtgaagaag cgatcatcaa aagccccatt 900

ctgatgacgt actggaatac tccggccttt tgctatcaca aatcagctcg cggcgctaaa 960

attctgacca aacgtcagaa aaacaacctg catacccaga tcaaattctg tcctggtggt 1020

tctgacgatg gttatctgtc aggtcgcttt tgtgcgtggc tgatgggata cgatgatgaa 1080

tacctggggg atcttatgca ctatgaattc 1110

## Description

### Technical Field

The present invention relates to a novel DNA methyltransferase.

### Background Art

DNA methyltransferases (MTases) are enzymes inducing methylation, which is one of the representative epigenetic modifications of DNA, and are also useful tools for epigenome analysis. DNA methylation at position 5 of cytosine (DNA cytosine 5-methylation (5mC)) is widely observed in the genomic DNA of living organisms.

However, as to DNA cytosine 5-methylation, MTases identified so far (Non-patent Documents 1 to 4) have a drawback in that their recognition sequences overlap with a CG dinucleotide sequence which is a recognition sequence for intrinsic DNA methylation (endogenous methylation) in mammalian cells, and also have a problem in that the number of bases in their recognition sequences is too long for fine epigenetic mapping.

### Prior Art Documents

[Non-patent Document 1] Renbaum, P. et al., Cloning, characterization, and expression in Escherichia coli of the gene coding for the CpG DNA methylase from Spiroplasma sp. strain MQ1(M.SssI). Nucleic acids research, 1990. 18(5): p. 1145-1152.
[Non-patent Document 2] Xu, M. et al., Cloning, characterization and expression of the gene coding for a cytosine-5-DNA methyltransferase recognizing GpC. Nucleic Acids Res, 1998. 26(17): p. 3961-3966. (which is the same as Reference 21 described later)
[Non-patent Document 3] Zhang, B. et al., The M.AluI DNA-(cytosine C5)-methyltransferase has an unusually large, partially dispensable, variable region. Nucleic Acids Res, 1993. 21(4): p. 905-911.
[Non-patent Document 4] Vladimir S Dedkov et al., Cloning and Study of New DNA Methyltransferase M.FatI Modifying Cytosine in a Recognition Site CATG. Research Journal of Pharmaceutical, Biological and Chemical Sciences, 2015. 6(6): p. 1341-1348.

### Summary of the Invention

Under these circumstances, there has been a demand for the development of a DNA methyltransferase (MTase) that is capable of introducing methylation which is distinguishable from endogenous methylation, i.e., capable of recognizing and methylating a short context other than but equivalent to a CG dinucleotide sequence where endogenous methylation occurs, etc.

The present invention has been made in consideration of the above situation and aims to provide a protein (MTase) and a gene encoding the same, etc., as set forth below.
[1] A protein as set forth in (a), (b) or (c) below:
   (a) a protein modified to delete some amino acid residues including the N-terminal end of DNA methyltransferase M.CviQIX or M.CviPII and thereby acquire the ability to specifically recognize a CC dinucleotide sequence and 5-methylate the 5'-position cytosine residue in the sequence;
   (b) a protein comprising an amino acid sequence with deletion, substitution or addition of one or several amino acids in the amino acid sequence of the protein (a) and having the ability to specifically recognize a CC dinucleotide sequence and 5-methylate the 5'-position cytosine residue in the sequence; or
   (c) a protein comprising an amino acid sequence sharing an identity of 80% or more with the amino acid sequence of the protein (a) and having the ability to specifically recognize a CC dinucleotide sequence and 5-methylate the 5'-position cytosine residue in the sequence.
[2] A protein as set forth in (a), (b) or (c) below:
   (a) a protein having the amino acid sequence as set forth in SEQ ID NO: 6 or 8;
   (b) a protein comprising an amino acid sequence with deletion, substitution or addition of one or several amino acids in the amino acid sequence of the protein (a) and having the ability to specifically recognize a CC dinucleotide sequence and 5-methylate the 5'-position cytosine residue in the sequence; or
   (c) a protein comprising an amino acid sequence sharing an identity of 80% or more with the amino acid sequence of the protein (a) and having the ability to specifically recognize a CC dinucleotide sequence and 5-methylate the 5'-position cytosine residue in the sequence.
[3] A gene encoding the protein according to [1] or [2] above.
[4] A gene comprising DNA as set forth in (a), (b) or (c) below:
   (a) DNA comprising the nucleotide sequence as set forth in SEQ ID NO: 5 or 7;
   (b) DNA which is hybridizable under stringent conditions with DNA consisting of a nucleotide sequence complementary to the DNA (a) and which encodes a protein having the ability to specifically recognize a CC dinucleotide sequence and 5-methylate the 5'-position cytosine residue in the sequence; or
   (c) DNA which shares an identity of 80% or more with the DNA (a) and which encodes a protein having the ability to specifically recognize a CC dinucleotide sequence and 5-methylate the 5'-position cytosine residue in the sequence.
[5] A recombinant vector comprising the gene according to [3] or [4] above.
[6] A transformant comprising the recombinant vector according to [5] above.
[7] A process for producing a protein having the ability to specifically recognize a CC dinucleotide sequence and 5-methylate the 5'-position cytosine residue in the sequence, which comprises the steps of:
   culturing the transformant according to [6] above; and
   collecting the protein from the resulting cultured material.
[8] A method for epigenome analysis, which comprises using the protein according to [1] or [2] above or a protein produced by the process according to [7] above.

### Effects of the Invention

The present invention enables the provision of a protein (MTase) that is capable of introducing methylation which is distinguishable from intrinsic DNA methylation (endogenous methylation) in mammalian cells, more particularly capable of specifically recognizing a "CC dinucleotide sequence" and 5-methylating the 5'-position cytosine residue in the sequence, as well as a gene encoding the same, etc. The protein (MTase) according to the present invention is useful in terms of theoretically achieving, e.g., a method for epigenome analysis (epigenome mapping) which allows simultaneous profiling of endogenous methylation and any other epigenomes.

### Brief Description of the Drawings

Figure 1 illustrates the scheme of plasmid vector pCZS.
Figure 2 illustrates a dual-affinity purification strategy used to purify candidate MTases.
   A. Structure of the expressed protein.
   B. Proteins purified by HisTrap purification and by dual-affinity purification combined with HisTrap and StrepTrap purification. M.CviPI is taken here as an example of MTase.
   C. Purified M.CviPI was assayed for its DNA methylation activity using the methylation-sensitive restriction enzyme HaeIII. Unmethylated lambda DNA (Promega) was used as substrate DNA. As a control, a lane was provided where M.CviPI purchased from NEB was used.

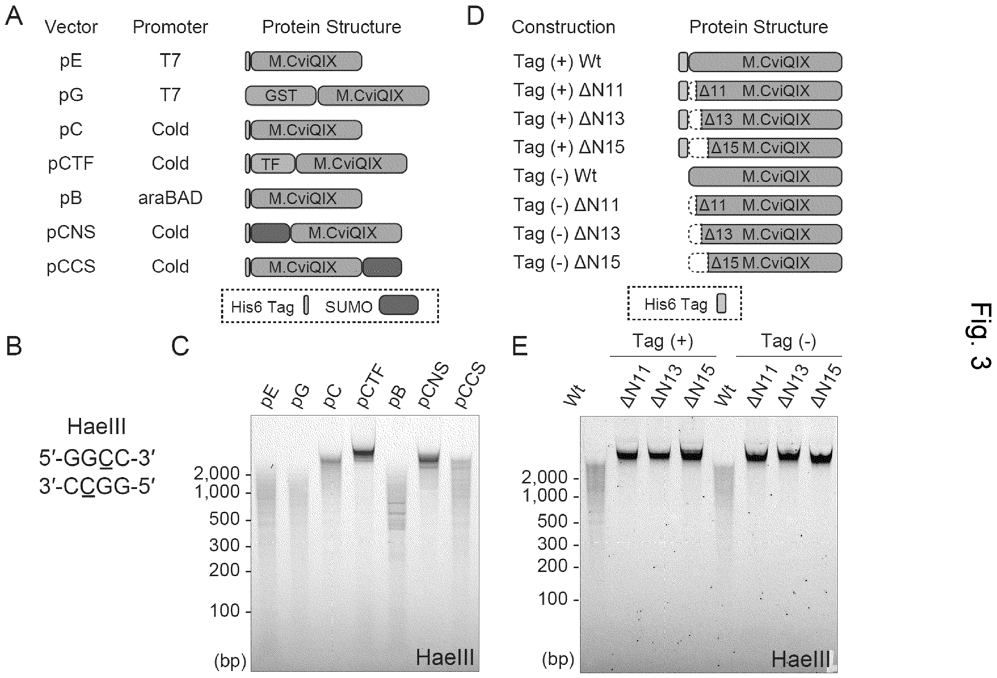

Figure 3 illustrates that N-terminal extension of M.CviQIX has an inhibitory effect against DNA methylation.
   A. Scheme illustrating recombinant protein structures of M.CviQIX.
   B. Recognition sequence of the restriction enzyme HaeIII. The recognition sequence contains CC dinucleotide. If the first C has been 5-methylated, HaeIII cannot cleave the substrate DNA.
   C. Results obtained when genomic DNAs extracted from bacterial cells expressing various M.CviQIX proteins as set forth in A were digested with HaeIII and analyzed on E-Gel Ex.
   D. Scheme illustrating the structures of various M.CviQIX protein constructs. Tag denotes the vector-derived sequence.
   E. Genomic DNA digestion of bacterial cells using the constructs as set forth in D.

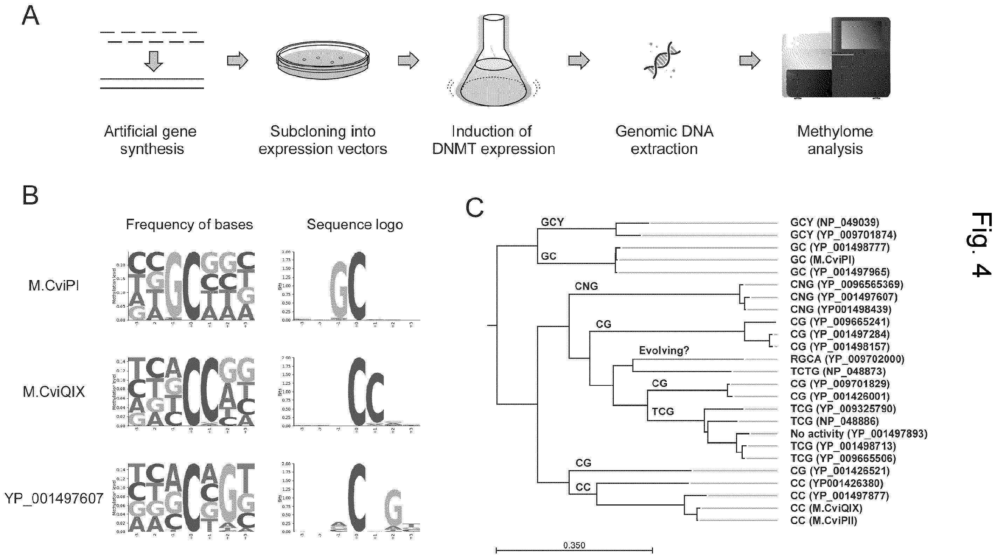

Figure 4 illustrates an experimental strategy to identify the recognition sequences of candidate MTases.
   A. Scheme used.
   B. Examples of identified sequence motifs.
   C. A phylogenetic tree was drawn based on the amino acid sequence similarity. The determined recognition sequences are arranged according to the gene names. The common recognition sequence of each clade is annotated at the top of the clade.

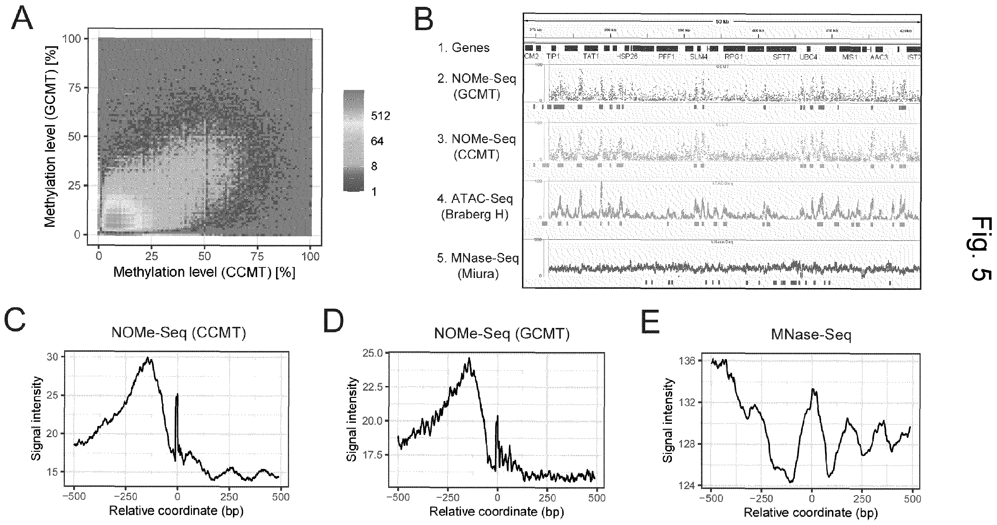

Figure 5 illustrates the results of NOMe-Seq of fixed yeast nuclei.
   A. Comparison of methylation levels between CCMT-treated nuclei and GCMT-treated nuclei. Bin size is 10 bp.
   B. Yeast genes (Track 1), DNA methylation levels at GC sites (Track 2, NOMe-Seq using GCMT) and CC sites (Track 3, NOMe-Seq using CCMT), read coverage of ATAC-Seq (Track 4, SRR12926697) (Reference 25), and read coverage of MNase-Seq (Track 5, SRR6729489) (Reference 26).
   C-E. Aggregation plots around the initiation codons of yeast genes. DNA methylation signals at CC (C) and GC (D) sites, and MNase-Seq (E) (SRR6729489) are drawn for illustrative purposes.

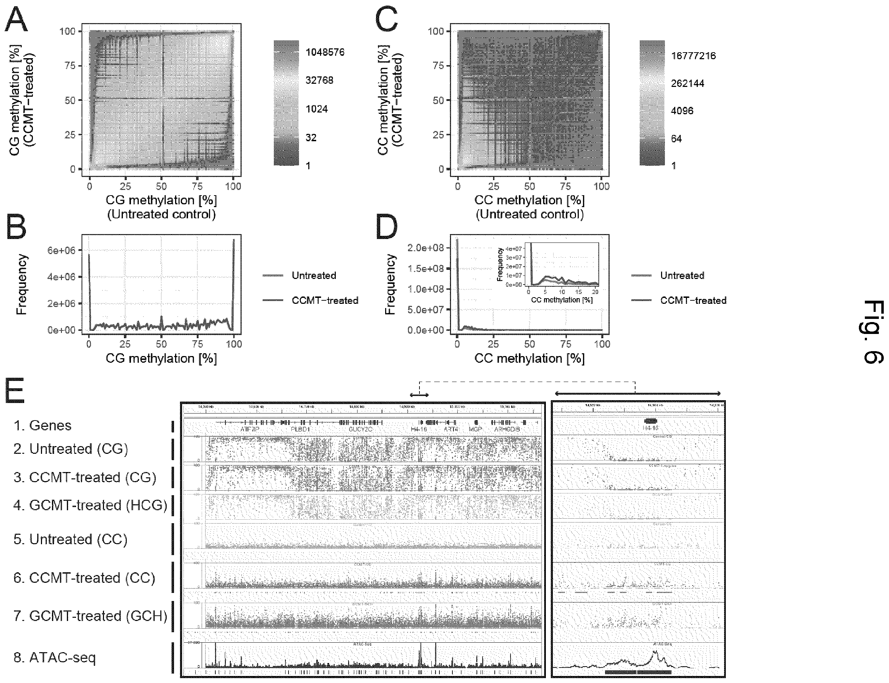

Figure 6 illustrates the results of NOMe-Seq of IMR-90 cells.
   A-D. Comparisons of DNA methylation levels between CCMT-treated nuclei and untreated nuclei. Endogenous DNA methylation at CG sites (A and B) and methylation at CC sites introduced via CCMT *in vitro* (C and D) are as set forth.
   E. Genome browser screenshots illustrating human genes (Track 1), intrinsic methylation levels (Tracks 2, 3 and 4 for untreated, CCMT-treated and GCMT-treated, respectively), MTase-induced methylation (Tracks 5, 6 and 7 for untreated, CCMT-treated and GCMT-treated, respectively), and read coverage of ATAC-Seq (SRR7765313) (Track 8). The right panel is an enlarged view of the indicated location in the left panel. For the tracks illustrating MTase-induced methylation patterns (Tracks 5, 6 and 7), peaks detected with macs2 are indicated at the bottom of each track.

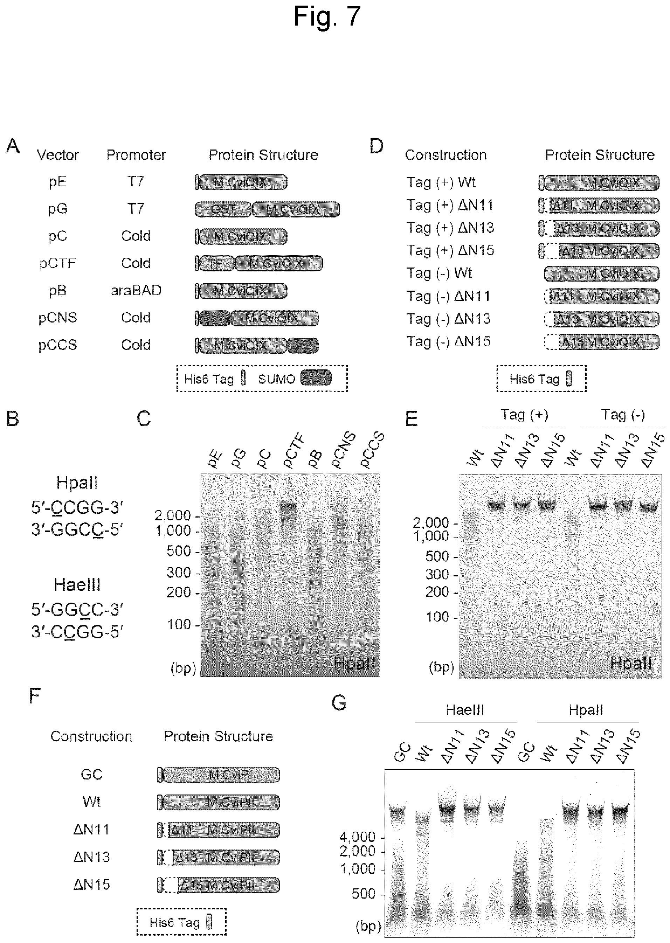

Figure 7 illustrates an attempt to express M.CviQIX and M.CviPII from several expression vectors.
   A. Scheme of constructs used for the expression of M.CviQIX.
   B. Recognition sequences of restriction enzymes used to study the methylation status of genomic DNA. Once the underlined C has been methylated, the activity of the enzymes will be inhibited.
   C. Analysis of the methylation status of the genomic DNAs extracted from *E*. *coli* cells induced to express M.CviQIX from the constructs as set forth in A.
   D. Scheme of N-terminal deletion mutants of M.CviQIX. A cold-shock promoter-based construct was used.
   E. Digestion assay of genomic DNAs extracted from *E coli* cells carrying the constructs as set forth in D.
   F. Deletion mutants of M.CviPII were compared.
   G. Restriction digestion assay of genomic DNAs extracted from *E. coli* cells carrying the constructs as set forth in F.

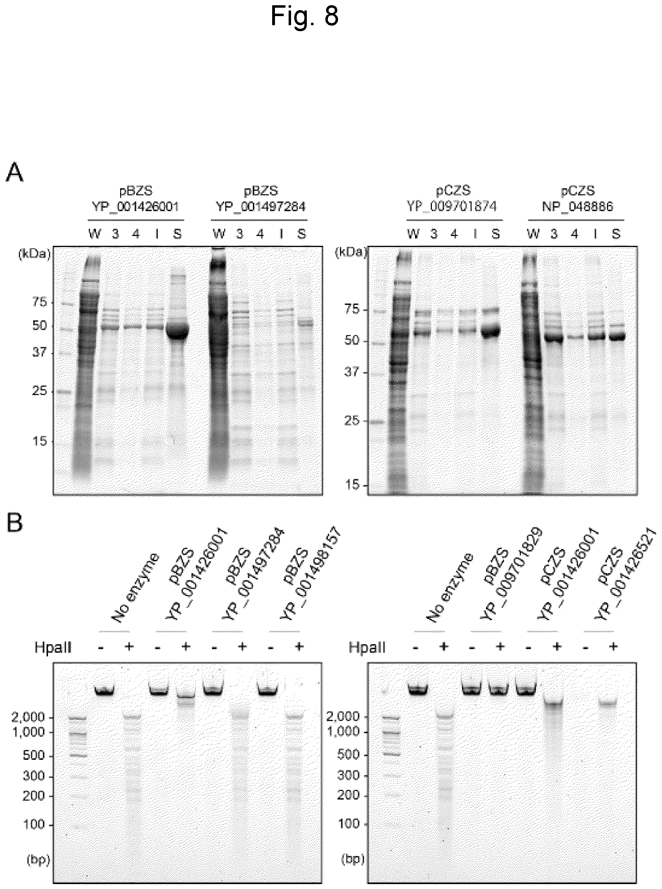

Figure 8 illustrates the purification of MTases and their activity.
   A. Representative SDS-PAGE gel images for analysis of protein purification. W: whole-cell extract; 3 and 4: 3rd and 4th fractions of HisTrap column purification, respectively; I: input of StrepTrap purification; and S: purified protein.
   B. Analysis of MTase activity of purified proteins. Unmethylated lambda DNA was treated with the purified MTases, digested with the methylation-sensitive restriction enzyme HpaII, and analyzed on E-Gel Ex.

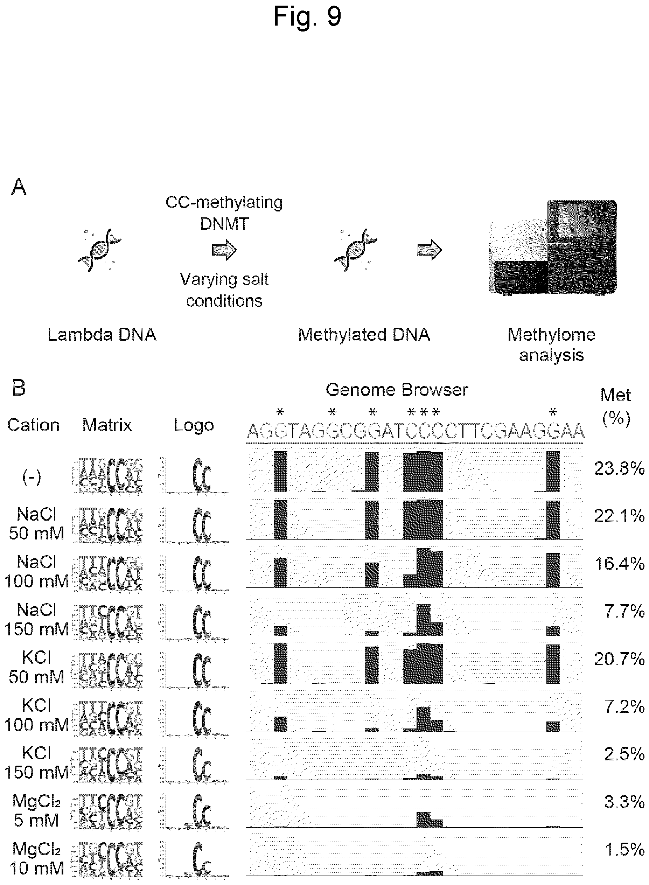

Figure 9 illustrates the results examined for the optimum salt concentration for CCMT.
   A. Strategy used for analysis.
   B. Methylation levels observed at the salt concentrations used.

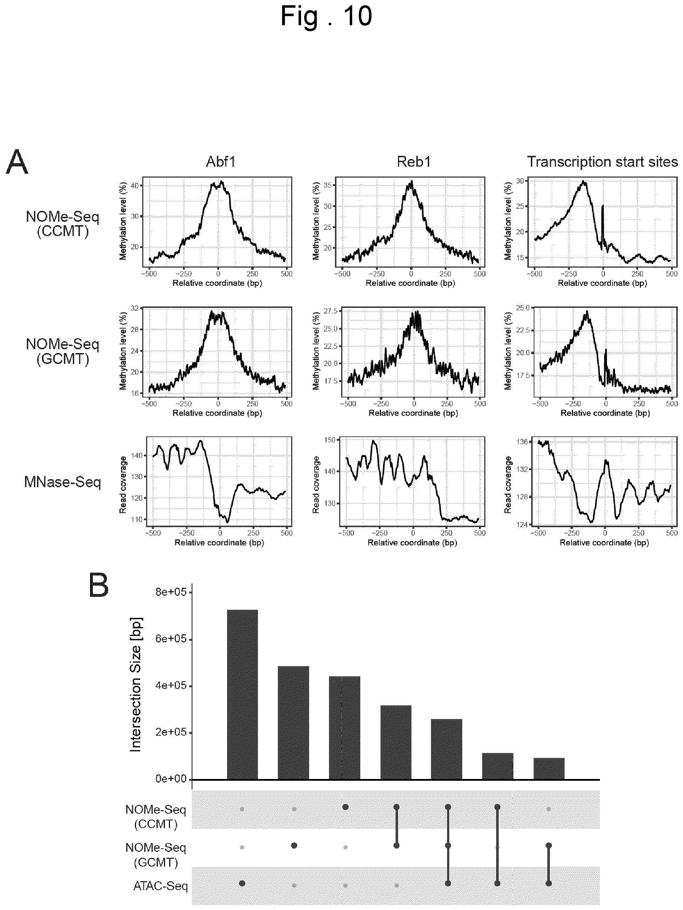

Figure 10 illustrates the analysis results of fixed budding yeast nuclei by GCMT- and CCMT-based NOMe-Seq.
   A. Aggregation plots for methylation levels of GCMT- and CCMT-based NOMe-Seq, and MNase-seq.
   B. Venn diagram illustrating the overlapping peaks identified by GCMT- and CCMT-based NOMe-Seq, ATAC-seq, and MNase-seq.

### Description of Embodiments

The present invention will be described in detail below. The scope of the present invention is not limited to the following descriptions, and any embodiments other than those illustrated below may also be carried out with appropriate modifications without prejudice to the spirit of the invention.

This specification incorporates the specification of Japanese Patent Application No. 2022-087994 (filed on May 30, 2022) in its entirety, based on which the present application claims priority.

All publications cited herein, including prior art documents, patent gazettes and other patent documents, are incorporated herein by reference.

As used herein, the term "DNA methyltransferase" and the term "MTase" are synonymous with each other.

It should be noted that detailed information for the references appearing in the following descriptions are listed at the end of this specification.

### 1. Summary and technical background according to the present invention

DNA cytosine 5-methylation (5mC) is widely observed in the genomic DNA of living organisms and forms one of the major epigenetic modifications. Classically, in mammalian cells, increased levels of 5mC were considered to lead to gene silencing, whereas the levels of gene body methylation are shown to positively correlate with the expression levels of genes (References 1-4). Thus, the distribution pattern of 5mC is cell type-specific, and the genome-wide distribution of 5mC or methylome has always attracted much attention of researchers.

Cytosine 5-methylation in mammalian cells depends on three DNA methyltransferases (MTases), i.e., DNMT1, DNMT3A and DNMT3B (References 5-7). DNMT1 is a maintenance DNA methyltransferase that introduces methylation into hemimethylated CG dinucleotides. On the other hand, DNMT3A and DNMT3B are *de novo* methyltransferases that introduce DNA methylation into non-methylated CG sites. Although some exceptions are known for pluripotent stem cells, oocytes, neurons and glial cells, DNA methylation on non-CG sites rarely occurs in normal somatic tissues (References 8 and 9). Thus, it is commonly accepted that 5mC is introduced almost exclusively into CG dinucleotides, and cytosines in contexts other than CG dinucleotides are unmethylated in most mammalian cells.

The deposition of 5mC in the genome is closely related to other epigenetic modifications. For example, 5mC is depleted in gene promoters, and trimethylation of lysine 4 of histone H3 (H3K4me3) is enriched (Reference 10). Another example is di- and tri-methylation of histone H3 lysine 36 (H3K36me2 and H3K36me3), and their deposition in the genome is positively related to transcriptional activity. H3K36me2 and H3K36me3 are enriched in gene bodies and recruit DNMT3A/B to introduce 5mC. Thus, these modifications support gene body methylation (References 11 and 12). Because many epigenetic modifications are mutually related, simultaneous detection of DNA methylation with other epigenetic markers on the same DNA molecule would stimulate the motivation for measurement, and the establishment of such measurement would shift epigenetic measurement to the next stage. However, most current methods for detecting epigenomes are not suitable for simultaneous detection.

For example, considering with chromatin immunoprecipitation followed by sequencing (ChIP-Seq) (References 13 and 14), which is the most commonly used technique for detecting epigenomes, in ChIP-Seq, the chromatin is first fragmented, the target epigenome on the fragmented chromatin is then immunoprecipitated, and DNA bound to the epigenome is sequenced. Since ChIP-Seq is based on chromatin fragmentation, the proximally located epigenomes on different nucleosomes should be separated in this first fragmentation step. Thus, measuring the relationship between two epigenetic modifications on a single DNA molecule is not practical with ChIP-Seq. Thus, fragmentation-based procedures are not suitable for measuring multiple epigenetic modifications simultaneously on a single DNA molecule.

However, there are some fragmentation-free procedures for epigenomic detection. For example, a DNA methyltransferase (MTase) can be used as a probe to measure chromatin accessibility.

Nucleosome occupancy and methylome sequencing (NOMe-Seq) is one of the earliest methods to measure chromatin accessibility with MTases (Reference 15), and NOMe-Seq has recently been combined with a nanopore sequencer (Reference 16). The same principle has recently been applied to Fiber-seq (Reference 17), which is a combination of non-specific adenine N-6 MTase M.EcoGII and a nanopore sequencer. Although chromatin accessibility is intended for the measurement of DNA-protein interactions of unspecified proteins, interactions between DNA and specific proteins can also be detected with MTases. One of the representative techniques is DamID (Reference 18). In DamID, dam methyltransferase fused with a protein of interest is expressed in target cells, and specific interactions of the fused protein with DNA can be measured by detecting DNA methylation introduced at the N-6 position of adenosine. In the original DamID report (Reference 18), methylation signals were detected using a methylation-sensitive restriction enzyme, but this is not an absolute requirement for detecting DNA methylation signals. Indeed, there are also cases where DNA methylation introduced with DamID was detected using a single-molecule sequencer. Moreover, DiMeLo-Seq for introducing adenine N-6 methylation in the proximity of a specific epigenomic modification has also been established, and the demand for (epigenomic) detection techniques using MTases is further increased.

Thus, MTases allow fragmentation-free and simultaneous detection of multiple epigenomes on a single DNA molecule.

For epigenetic analysis based on MTases, the resolution is primarily determined by the frequency of the recognized sequences in the genome, and the length of the recognized sequence determines the frequency of sequences. Since one of the most basic epigenome units, nucleosomes, are wrapped with approximately 150 base pairs (bp) of genomic DNA, the resolution required for epigenome mapping should be higher than 150 bp. Thus, the recognition sequence must be 3 bp (64 bp resolution) or less to achieve sufficient resolution for epigenetic measurement. Many MTases have been identified from bacterial and viral sources, and non-specific adenine N-6 MTases, such as M. EcoGII, have achieved fine mapping of chromatin accessibility (Reference 17). However, when focusing on DNA cytosine C-5 MTases, MTases that can be used as probes for fine epigenome mapping are limited to only three: M. SssI (CG), M. CviPI (GC) and M. CviPII (CCD) (the target cytosines are underlined) (References 19 and 20). Further, due to the intrinsic DNA methylation (endogenous methylation) of CG dinucleotides in mammalian cells, a problem arises from the existence of sequences fully (M.SssI) or partially (M.CviPI) overlapping with CG dinucleotides. Thus, there has been a demand for the development and identification of cytosine C-5 MTases that never overlap with CG dinucleotides and has a short recognition sequence.

The inventor of the present invention has found a systematic strategy for identifying cytosine C-5 MTases with short recognition sequences, and have succeeded in identifying the MTases of six novel recognition sequences. Moreover, the inventor of the present invention has developed a novel MTase by focusing on MTases capable of recognizing CC dinucleotide among the identified MTases, because the non-overlapping specificity with CG dinucleotide is thought to be beneficial for epigenome mapping in mammalian cells. More particularly, this novel MTase is an MTase that is capable of specifically recognizing a CC dinucleotide sequence and 5-methylating the 5'-position cytosine residue in the sequence. Such a novel MTase capable of recognizing and methylating CC dinucleotide, which is a context composed of only two nucleotides and not overlapping with CG where endogenous methylation occurs, has not yet been reported or established.

### 2. Protein

The protein according to the present invention is a protein having functions as a DNA methyltransferase (MTase). Particularly, the protein according to the present invention is a protein having the ability to specifically recognize a CC dinucleotide sequence and 5-methylate the 5'-position cytosine residue in the sequence. More particularly, the protein according to the present invention is a protein modified to delete some amino acid residues including the N-terminal end of an MTase, M.CviQIX or M.CviPII, and thereby have (or acquire) the above ability (i.e., the ability to 5-methylate the 5'-position cytosine residue in the CC dinucleotide sequence).

M.CviQIX is an MTase which has been considered to be derived from *Paramecium bursaria* Chlorella virus NY2A, and its amino acid sequence is as set forth in SEQ ID NO: 2. It should be noted that the nucleotide sequence encoding this amino acid sequence is not limited to, but is illustrated by, the nucleotide sequence as set forth in SEQ ID NO: 1.

M.CviPII is an MTase derived from Chlorella virus, and its amino acid sequence is as set forth in SEQ ID NO: 4. It should be noted that the nucleotide sequence encoding this amino acid sequence is not limited to, but is illustrated by, the nucleotide sequence as set forth in SEQ ID NO: 3.

It should be noted that the MTases M.CviQIX and M.CviPII are each one of many conventionally known MTases, and are known to be capable of specifically recognizing a "CCD trinucleotide sequence" (wherein D is A, G or C) and 5-methylating the 5'-position cytosine residue in this sequence. In general, those skilled in the art would generally have no rational reason for attempting to further modify such an MTase capable of recognizing and methylating a relatively short sequence of CCD trinucleotide. Moreover, even when attempting to modify an MTase capable of recognizing and methylating a shorter dinucleotide sequence, there is no rational reason leading to a means intended to delete amino acid residues including the N-terminal end of the MTase.

A protein modified to delete some amino acid residues including the N-terminal end of the MTase M.CviQIX is not limited to, and may be, for example, a protein modified to delete 11 amino acid residues, 13 amino acid residues or 15 amino acid residues from the N-terminal end of this MTase. Among them, a protein modified to delete 15 amino acid residues is preferred. The amino acid sequence of this protein modified to delete 15 amino acid residues from the N-terminal end of M.CviQIX is the amino acid sequence as set forth in SEQ ID NO: 6 (it should be noted that the nucleotide sequence encoding this amino acid sequence is as set forth in SEQ ID NO: 5). For this reason, the protein according to the present invention is also preferably illustrated by a protein having the amino acid sequence as set forth in SEQ ID NO: 6.

A protein modified to delete some amino acid residues including the N-terminal end of the MTase M.CviPII is not limited to, and may be, for example, a protein modified to delete 11 amino acid residues, 13 amino acid residues or 15 amino acid residues from the N-terminal end of this MTase. Among them, a protein modified to delete 15 amino acid residues is preferred. The amino acid sequence of this protein modified to delete 15 amino acid residues from the N-terminal end of M.CviPII is the amino acid sequence as set forth in SEQ ID NO: 8 (it should be noted that the nucleotide sequence encoding this amino acid sequence is as set forth in SEQ ID NO: 7). For this reason, the protein according to the present invention is also preferably illustrated by a protein having the amino acid sequence as set forth in SEQ ID NO: 8.

The protein according to the present invention is not limited to, and is also preferably illustrated by proteins (so-called mutant proteins) comprising amino acid sequences in which one or several amino acids have been deleted, substituted or added in the amino acid sequences of the various proteins mentioned above in this section and having the ability to specifically recognize a CC dinucleotide sequence and 5-methylate the 5'-position cytosine residue in the sequence.

As intended here, the above "amino acid sequence in which one or several amino acids have been deleted, substituted or added" is preferably an amino acid sequence in which, for example, 1 to around 10 amino acids, preferably 1 to several amino acids, 1 to 5 amino acids, 1 to 4 amino acids, 1 to 3 amino acids, 1 to 2 amino acids, or 1 amino acid has been deleted, substituted or added. For introduction of mutations such as deletion, substitution or addition, it is possible to use a kit for mutation introduction based on site-directed mutagenesis, as illustrated by GeneTailor^{™} Site-Directed Mutagenesis System (Invitrogen) and TaKaRa Site-Directed Mutagenesis System (e.g., Prime STAR^{®} Mutagenesis Basal kit, Mutan^{®}-Super Express Km; Takara Bio Inc., Japan), etc. Likewise, whether the above-mentioned mutation of deletion, substitution or addition has been introduced can be confirmed using various amino acid sequencing techniques, as well as X ray- and NMR-based structural analysis techniques, etc.

Moreover, the protein according to the present invention is not limited to, and is also preferably illustrated by, proteins (so-called mutant proteins) that comprise amino acid sequences having an identity (homology) of 80% or more to the amino acid sequences of the various proteins mentioned above and that have the ability to specifically recognize a CC dinucleotide sequence and 5-methylate the 5'-position cytosine residue in the sequence.

As intended here, the above identity is preferably 85% or more, 90% or more, 95% or more, 96% or more, 97% or more, 98% or more, or 99% or more.

Mutant proteins as mentioned above may also be prepared through genetic engineering procedures using a gene encoding the amino acid sequences of the protein.

In the context according to the present invention, the term "specifically recognize a CC dinucleotide sequence" is intended to mean that only a dinucleotide composed of two consecutive cytosine (C) nucleotides can be recognized. The term "5-methylate the 5'-position cytosine residue in the CC dinucleotide sequence" is intended to mean methylating the carbon of the 5'-position of the cytosine residue at the 5th end of the above recognized CC, whereby the 5'-position cytosine residue, resulted in 5-methylcytosine or 5-methylcytidine (5mC). Such methylation activity may be detected, measured and/or evaluated, for example, by techniques to examine the presence or absence of DNA cleavage with HaeIII (which recognizes GGCC) or HpaII (which recognizes CCGG) whose cleavage activity is known to be inhibited by the presence of 5mC, or by bisulfite sequencing techniques, etc.

The protein according to the present invention may be composed of naturally-occurring peptides linked in combination or may be obtained artificially by chemical synthesis, is not limited thereto.

A naturally-occurring protein may be directly obtained from a naturally-occurring product by known recovery and purification procedures, or may be obtained by known gene recombination technology as follows: a gene encoding the protein is incorporated into any of various expression vectors or the like, introduced the vector into cells and expressed therein, followed by known recovery and purification procedures. Alternatively, the protein may be produced by a cell-free protein synthesis system using a commercially available kit, e.g., a reagent kit PROTEIOS^{™} (Toyobo Co., Ltd., Japan) or TNT^{™} System (Promega), a synthesizer PG-Mate^{™} (Toyobo Co., Ltd., Japan) or RTS (Roche Diagnostics), etc., followed by known collection and purification procedures, and is not limited thereto.

On the other hand, a chemically synthesized protein may be obtained by known protein synthesis techniques. Examples of synthesis techniques include azide method, acid chloride method, acid anhydride method, mixed acid anhydride method, DCC method, active ester method, carboimidazole method and oxidation-reduction method, etc. Moreover, the synthesis may be accomplished by applying either solid phase synthesis or liquid phase synthesis. A commercially available protein synthesizer may also be used for this purpose. After synthesis reaction, the protein may be purified using known purification techniques (e.g., chromatography) in combination.

The present invention may include derivatives of the above various proteins according to the present invention together with or in place of these proteins. Such derivatives are intended to encompass all derivatives which may be prepared originating from these proteins, as illustrated by those in which some of the constituent amino acids are replaced with unnatural amino acids and those in which some of the constituent amino acids (mainly their side chains) are chemically modified, etc.

Moreover, according to the present invention, salts of the above various proteins and/or derivatives thereof may be included together with or in place of these proteins and/or derivatives thereof. Such salts are preferably physiologically acceptable acid addition salts or basic salts. Examples of acid addition salts include salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid). Examples of basic salts include salts with inorganic bases (e.g., sodium hydroxide, potassium hydroxide, ammonium hydroxide, magnesium hydroxide) or salts with organic bases (e.g., caffeine, piperidine, trimethylamine, pyridine).

These salts may be prepared using appropriate acids such as hydrochloric acid or using appropriate bases such as sodium hydroxide. For example, these salts may be prepared by treatment in water or in a liquid containing an inert water-miscible organic solvent (e.g., methanol, ethanol or dioxane) using standard protocols.

### 2. Recombinant gene

A gene encoding the protein according to the present invention as mentioned above is not limited to, but is illustrated by, a gene comprising DNA as set forth in (a), (b) or (c) below. Genes comprising these DNAs may consist only of these DNAs or may comprise, in addition to these DNAs, known nucleotide sequences (e.g., a transcription promoter, an SD sequence, a Kozak sequence, a terminator) required for gene expression and/or recognition sequences for various restriction enzyme sites, without being limited thereto.
(a) DNA comprising the nucleotide sequence as set forth in SEQ ID NO: 5 or 7.
(b) DNA which is hybridizable under stringent conditions with DNA consisting of a nucleotide sequence complementary to the above DNA (a) and which encodes a protein having the ability to specifically recognize a CC dinucleotide sequence and 5-methylate the 5'-position cytosine residue in the sequence.
(c) DNA which shares an identity of 80% or more with the above DNA (a) and which encodes a protein having the ability to specifically recognize a CC dinucleotide sequence and 5-methylate the 5'-position cytosine residue in the sequence.

In the above DNA (a), the nucleotide sequence as set forth in SEQ ID NO: 5 is a nucleotide sequence encoding an amino acid sequence with deletion of N-terminal 15 residues in the amino acid sequence (SEQ ID NO: 2) of the MTase M.CviQIX, as mentioned above. Likewise, the nucleotide sequence as set forth in SEQ ID NO: 7 is a nucleotide sequence encoding an amino acid sequence with deletion of N-terminal 15 residues in the amino acid sequence (SEQ ID NO: 4) of the MTase M.CviPII.

The above DNA (b) may be obtained from a cDNA library or a genomic library by known hybridization techniques such as colony hybridization, plaque hybridization and Southern blotting in which the above DNA (a) or DNA consisting of a nucleotide sequence complementary thereto, or a fragment thereof is used as a probe. As such a library, a library prepared in a known manner or a commercially available cDNA or genomic library may be used for this purpose and is not limited thereto.

As to detailed procedures for hybridization techniques, reference may be made, as appropriate, to Molecular Cloning, A Laboratory Manual 4th ed., Cold Spring Harbor Laboratory Press (1989), etc.

The "stringent conditions" in hybridization techniques refer to washing conditions after hybridization, and are intended to mean, for example, conditions where the salt concentration in buffer is 15 to 330 mM and the temperature is 25°C to 65°C, preferably conditions where the salt concentration is 15 to 150 mM and the temperature is 45°C to 55°C, as specifically illustrated by conditions where the salt concentration is 80 mM and the temperature is 50°C, etc. Further, in addition to such conditions of the salt concentration and the temperature, various other conditions including the probe concentration, the probe length, the reaction time and so on may also be taken into consideration to determine, as appropriate, the conditions required to obtain the above DNA (b).

The above DNA (c) is DNA having an identity (homology) of 80% or more to the above DNA (a), as preferably illustrated by DNA having an identity of 85% or more, 90% or more, 95% or more, 96% or more, 97% or more, 98% or more, or 99% or more to the above DNA (a). This DNA is preferred for use as the above hybridizable DNA (b).

Mutant DNA such as the above DNA (c) may be prepared, for example, according to site-directed mutagenesis as described in Molecular Cloning, A Laboratory Manual 4th ed., Cold Spring Harbor Laboratory Press (1989), Current Protocols in Molecular Biology, John Wiley & Sons (1987-1997), etc. Particularly, mutant DNA may be prepared by known techniques (e.g., Kunkel method or Gapped duplex method) using a kit for introducing mutation based on site-directed mutagenesis, and preferred examples of such a kit include QuickChange^{™} Site-Directed Mutagenesis Kit (Stratagene), GeneTailor^{™} Site-Directed Mutagenesis System (Invitrogen), TaKaRa Site-Directed Mutagenesis System (e.g., Mutan-K, Mutan-Super Express Km; Takara Bio Inc., Japan), etc.

Alternatively, using PCR primers designed such that a missense mutation is introduced to give nucleotides representing a codon for a desired amino acid, DNA comprising a nucleotide sequence encoding wild-type MTG may be used as a template in PCR under appropriate conditions to prepare mutant DNA. Any DNA polymerase may be used in PCR, but preferred is a highly accurate DNA polymerase, as preferably illustrated by Pwo DNA polymerase (Roche Diagnostics), Pfu DNA polymerase (Promega), platinum Pfx DNA polymerase (Invitrogen), KOD DNA polymerase (Toyobo Co., Ltd., Japan), KOD-plus-polymerase (Toyobo Co., Ltd., Japan), etc. Reaction conditions for PCR may be determined as appropriate depending on the optimum temperature of the DNA polymerase to be used, the length and type of DNA to be synthesized, etc. For example, cycle conditions are preferably set as follows: 20 to 200 cycles in total of "90°C to 98°C for 5 to 30 seconds (thermal denaturation and dissociation) → 50°C to 65°C for 5 to 30 seconds (annealing) → 65°C to 80°C for 30 to 1200 seconds (synthesis and elongation)."

As to the above DNA (b) or (c), for example, particularly preferred is DNA consisting of a nucleotide sequence such that this nucleotide sequence is not completely the identical as that of the above DNA (a) but the amino acid sequence translated therefrom is completely the same as that from the above DNA (a) (i.e., DNA engineered to comprise a silent mutation(s) in the above DNA (a)).

For description about "the ability to specifically recognize a CC dinucleotide sequence and 5-methylate the 5'-position cytosine residue in the sequence" in the above DNA (b) or (c), the descriptions in the preceding section may be applied as appropriate.

The gene encoding the protein according to the present invention has no particular limitation on its codons corresponding to individual amino acids translated therefrom, and may comprise DNA representing codons commonly used (preferably codons frequently used) in humans or other mammals after transcription or may comprise DNA representing codons commonly used (preferably codons frequently used) in microorganisms (e.g., *E. coli* or yeast) or plants, etc., after transcription.

Moreover, if the protein according to the present invention comprises a linker sequence as mentioned above, the gene encoding the protein according to the present invention may comprise DNA encoding the amino acid sequence of the linker sequence.

### 3. Recombinant vector and transformant

To express the protein according to the present invention, the gene according to the present invention as mentioned above should first be incorporated into an expression vector to construct a recombinant vector. In this case, the gene to be incorporated into the expression vector may optionally be linked in advance in its upstream with a transcription promoter, an SD sequence (when prokaryotic cells are used as a host) and a Kozak sequence (when eukaryotic cells are used as a host) and linked in advance in its downstream with a terminator, and may also be linked in advance with an enhancer, a splicing signal, a poly-A addition signal, a selective marker, etc. It should be noted that individual elements required for gene expression including a transcription promoter as mentioned above may be contained initially in the gene, or alternatively, those contained originally in the expression vector, if any, may be used. There is no particular limitation on the mode of use of each element.

For incorporating the gene into the expression vector, various known techniques based on gene recombination technology may be used, including those using restriction enzymes, those using topoisomerases, etc. Moreover, the expression vector is not limited in any way as long as it may carry the gene encoding the protein according to the present invention, as illustrated by plasmid DNA, bacteriophage DNA, retrotransposon DNA, a retroviral vector, artificial chromosomal DNA, etc., and a vector suitable for host cells to be used may be selected as appropriate for use.

Then, the above recombinant vector thus constructed may be introduced into a host to obtain a transformant, and this transformant may be cultured to allow the expression of the protein according to the present invention. It should be noted that the term "transformant" in the context according to the present invention is intended to mean a host into which a foreign gene has been introduced, and examples include a host into which a foreign gene has been introduced by introduction of plasmid DNA or the like (transformation) and a host into which a foreign gene has been introduced by infection with various viruses and phages (transduction).

Such a host is not limited, and may be selected as appropriate as long as it allows the expression of the protein according to the present invention after introduction of the above recombinant vector, and examples include known hosts such as various animal cells (e.g., human and mouse cells), various plant cells, bacteria, yeast, plant cells, etc.

When animal cells are used as a host, examples include human fibroblasts, CHO cells, monkey cells COS-7, Vero, mouse L cells, rat GH3, human FL cells, etc. Alternatively, insect cells such as Sf9 cells and Sf21 cells may also be used.

When bacteria are used as a host, examples include *E. coli, Bacillus subtilis,* etc.

When yeast is used as a host, examples include *Saccharomyces cerevisiae, Schizosaccharomyces pombe,* etc.

When plant cells are used as a host, examples include tobacco BY-2 cells, etc.

Procedures for obtaining a transformant are not limited, and may be selected as appropriate in consideration of the combination of types of host and expression vector. Preferred examples include electroporation, lipofection, heat shock transfection, PEG transfection, calcium phosphate transfection, DEAE-dextran transfection, and infection with various viruses (e.g., DNA virus, RNA virus).

In the resulting transformant, the codon types of the gene contained in the recombinant vector are not limited, and may be either identical with or different from the codon types in the host actually used.

### 4. Process for protein production

The protein according to the present invention can be produced, particularly, by a process comprising a step of culturing the above transformant and a step of collecting a protein having the ability to specifically recognize a CC dinucleotide sequence and 5-methylate the 5'-position cytosine residue in the sequence from the resulting cultured material. As used here, the term "cultured material" is intended to mean a culture supernatant, cultured cells, cultured microorganisms, or a disruptant of the cells or microorganisms. The above transformant may be cultured in accordance with standard procedures used for host culture. The desired protein is accumulated in the above cultured material. According to the present invention, the above collection step may comprise the purification step of the protein.

As a medium used for the above culture, any of various known natural and synthetic media may be used as long as it contains host-assimilable sources of carbon, nitrogen, inorganic salts, etc., and allows efficient culture of the transformant.

During culture, the transformant may be cultured under selection pressure to avoid loss of the recombinant vector contained in the transformant and loss of the gene encoding the desired protein. Namely, when the selective marker used is a drug resistance gene, the corresponding drug may be added to the medium, while when the selective marker used is an auxotrophic complementary gene, the corresponding trophic factor may be removed from the medium.

In the case of culturing a transformant transformed with an expression vector using an inducible promoter as a promoter, a suitable inducer (e.g., IPTG) may be added to the medium when required.

Culture conditions for the transformant are not limited as long as they do not impair the productivity of the desired protein and the growth of the host. In general, culture may be conducted at 10°C to 40°C, preferably at 20°C to 37°C for 5 to 100 hours. pH adjustment may be accomplished by using an inorganic or organic acid, an alkaline solution, etc. Techniques used for culture include solid culture, static culture, shaking culture, aerobic spinner culture, etc.

After culture, when the desired protein is produced within microorganisms or cells, the microorganisms or cells may be disrupted to thereby collect the desired protein. Techniques for disruption of the microorganisms or cells include high pressure treatment with a French press or a homogenizer, ultrasonication, grinding treatment with glass beads, etc., enzymatic treatment with lysozyme, cellulase or pectinase, etc., freeze-thaw treatment, hypotonic solution treatment, and bacteriolysis induction treatment with phages. After disruption, the disrupted debris of the microorganisms or cells (including an insoluble cell extract fraction) may be removed, if necessary. Techniques for removal of the debris may be illustrated by centrifugation and filtration, etc. If necessary, a flocculating agent and/or a filter aid, etc., may be used to increase the removal efficiency of the debris. The supernatant obtained after removal of the debris is a soluble cell extract fraction and can be used as a crude protein solution.

Alternatively, when the desired protein is produced within microorganisms or cells, the microorganisms or cells per se may be collected by centrifugation, membrane separation or other techniques, and used directly without being disrupted.

On the other hand, when the desired protein is produced outside microorganisms or cells, the cultured solution may be used directly or may be treated by centrifugation, filtration or other techniques to remove the microorganisms or cells. Then, the desired protein may be collected from the cultured material, e.g., through extraction by ammonium sulfate precipitation, if necessary, and may further be isolated and purified by using dialysis and/or various chromatographic techniques (e.g., gel filtration, ion exchange chromatography, affinity chromatography), if necessary.

The production yield of the protein obtained upon culture of the transformant may be confirmed, for example, by SDS-PAGE (polyacrylamide gel electrophoresis) or other techniques in units, for example, per culture solution, per wet cell weight or dry cell weight, per crude enzyme solution protein, etc.

Moreover, in addition to a protein synthesis system as mentioned above using a transformant, the desired protein may also be produced using a cell-free protein synthesis system where no living cells are used.

Such a cell-free protein synthesis system is a system where a cell extract is used to synthesize a desired protein within an artificial container such as a test tube, etc. Moreover, cell-free protein synthesis systems available for this purpose also include a cell-free transcription system where DNA is used as a template to synthesize RNA.

In this case, the cell extract to be used is preferably derived from host cells described above. As a cell extract, it is possible to use, for example, an extract derived from eukaryotic cells or prokaryotic cells, more particularly an extract derived from CHO cells, rabbit reticulocytes, mouse L-cells, HeLa cells, wheat germ, budding yeast, *E. coli* or the like. It should be noted that these cell extracts may be concentrated or diluted before use, or may be used directly, without being limited thereto.

These cell extracts may be obtained, for example, by ultrafiltration, dialysis, polyethylene glycol (PEG) precipitation, etc.

Such cell-free protein synthesis may also be accomplished by using a commercially available kit. Examples include a reagent kit PROTEIOS^{™} (Toyobo Co., Ltd., Japan) or TNT^{™} System (Promega), a synthesizer PG-Mate^{™} (Toyobo Co., Ltd., Japan) or RTS (Roche Diagnostics), etc.

The desired protein produced by cell-free protein synthesis may be purified as mentioned above by chromatographic or other means which may be selected as appropriate.

### 5. Uses of protein

The present invention also includes a method for epigenome analysis (epigenome mapping), which comprises using the protein according to the present invention (including proteins obtainable by the production processes described in the preceding section).

Despite the increasing demand for MTases as tools for epigenome mapping, the available enzymes are limited, particularly for cytosine C-5 MTases having short recognition sequences. The protein according to the present invention is capable of recognizing and methylating a CC dinucleotide sequence which is distinct from (i.e., does not overlap with) a CG dinucleotide sequence where endogenous methylation occurs in mammalian cells. For this reason, the protein according to the present invention is useful as a new probe for epigenome analysis (epigenome mapping).

### Examples

The present invention will be further described in more detail by way of the following examples, although the present invention is not limited only to these examples.

### [Example 1]

### 1. Materials and methods

### Sequence analysis

The amino acid sequence of M. CviQIX was obtained from REBASE (http://rebase.neb.com/rebase/rebase.html) (References 19 and 20), while the amino acid sequence of M.CviPII was obtained from Reference 21.

### Artificial gene synthesis and subcloning into expression vectors

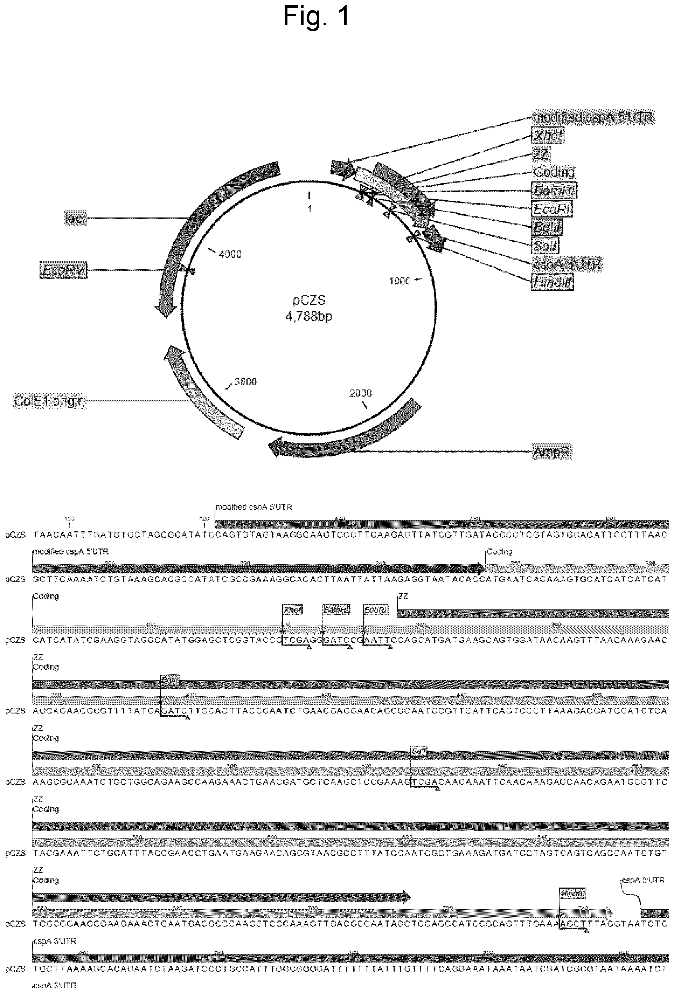

Genes encoding M.CviQIX and M.CviPII were synthesized with codon optimization for *E. coli* by Eurofin Genomics Inc. The genes were synthesized to have BamHI and EcoRI recognition sequences at the 5'- and 3'-terminal ends, respectively, with no internal BamHI and EcoRI recognition sequences (SEQ ID NOs: 9 and 10 (the nucleotide sequences of SEQ ID NOs: 9 and 10 are designed to have the above recognition sequences at the 5'- and 3'-terminal ends of the nucleotide sequences of SEQ ID NOs: 5 and 7, respectively)). These gene fragments were each subcloned into the BamHI-EcoRI site of pCZS (Figure 1). Genes for N-terminal deletion mutants of M.CviQIX and M.CviPII were prepare from the expression vectors carrying their respective full-length genes through inverse PCR and the subsequent circularization by Infusion cloning (Takara Bio Inc., Japan).

### Small-scale induction of MTases and bacterial genomic DNA extraction

The bacterial expression vector pCZS carrying the gene encoding M.CviQIX or M.CviPII was introduced into T7Express (New England Biolabs, Ipswich, MA). The cells were inoculated into 3 mL of LB medium (1% [w/v] tryptone, 0.5% [w/v] yeast extract, and 0.5% [w/v] sodium chloride) supplemented with 100 µg/mL of carbenicillin (Nacalai Tesque, Inc., Kyoto, Japan) and grown overnight at 37°C with vigorous shaking. 50 µL of each cell suspension was diluted with 3 mL of carbenicillin-supplemented LB medium, and the cells were cultured at 37°C for 3 hours with shaking. This cell suspension was cooled in ice-cold water for 30 minutes, and 3 µL of 1 M isopropyl β-D-1-thiogalactopyranoside (IPTG, Nacalai Tesque, Inc., Japan) was added to induce protein expression. The cells were collected by centrifugation and used for genomic DNA purification with a DNeasy Blood & Tissue Kit (Qiagen). The genomic DNA concentration was measured using a Qubit dsDNA BR assay kit and a Qubit fluorometer (Thermo Fisher Scientific, Waltham, MA, USA).

### Analysis of DNA methylation by restriction enzyme digestion

Genomic DNA (100 ng) was added to a 10 µL reaction containing 1× CutSmart Buffer and a restriction enzyme, HaeIII or HpaII. The reaction mixture was incubated at 37°C for 1 hour, followed by heating at 70°C for 10 minutes to inactivate the enzyme. After treatment with either of the above restriction enzymes, DNA was analyzed using an E-Gel Power SNAP system on a 2% E-Gel Ex gel (Thermo Fisher Scientific).

### Purification of recombinant MTases

The bacterial cells transformed with pCZS expressing the MTase-encoding gene was inoculated into 30 mL of LB medium containing 100 µg/mL carbenicillin, and cultured overnight at 37°C with vigorous shaking. The seed culture was transferred to a 2 L Erlenmeyer flask with baffles containing 1 L of carbenicillin-supplemented LB medium, and further cultured at 37°C for 4 hours with shaking. This flask was cooled in ice-cold water for 30 minutes, and 230 mg of IPTG was then added to induce protein expression. The flask was incubated overnight at 16°C with vigorous shaking. The cells were collected by centrifugation at 2500 × g for 15 minutes, and the collected cells were frozen at -80°C until use.

The bacterial cells were re-suspended in 20 mL of HisTrap buffer A (20 mM sodium phosphate, pH 7.5, 800 mM NaCl, 20 mM imidazole, 1 mM DTT, and 10% glycerol) containing a protease inhibitor cocktail (Nacalai Tesque, Inc., Japan) and disrupted through ultrasonication. The lysate was cleared by centrifugation at 15,000 × g for 15 minutes and filtered with a 0.45 µm syringe filter. The first round of affinity purification was performed with a 5 mL HisTrap HP column (Cytiva, Marlborough, MA) using HisTrap buffer A and HisTrap buffer B (20 mM sodium phosphate, pH 7.5, 800 mM NaCl, 200 mM imidazole, 1 mM DTT, 10% glycerol). A fraction containing the target protein was further purified with a 1 mL StrepTrap HP (Cytiva) using StrepTrap buffer A (100 mM Tris-HCl, pH 8.0, 150 mM NaCl, 1 mM EDTA, 1 mM DTT) and StrepTrap buffer B (100 mM Tris-HCl, pH 8.0, 150 mM NaCl, 1 mM EDTA, 1 mM DTT, and 2.5 mM d-desthiobiotin). Chromatographic purification was performed with an AKTA Start System (Cytiva). The dual-affinity purified protein was concentrated by ultrafiltration using an Amicon Ultra-4 30 K device (Millipore), and supplemented with bovine serum albumin (BSA) and glycerol to give final concentrations of 100 µg/mL and 50% (v/v), respectively, and then stored at -20°C until use.

### Budding yeast NOMe-Seq

S288C cells (NBRC1136, Biological Resource Center at the National Institute of Technology and Evaluation) were inoculated into 50 ml of YPD medium (1% yeast extract, 2% Bacto peptone, 2% dextrose) and cultured overnight at 30°C with shaking. The cell suspension was diluted with 1 L of YPD and further cultured at 30°C for 4 hours with shaking. The cells were collected by centrifugation at 2000 × g for 15 minutes, washed twice with 50 mL of water, and re-suspended in 20 mL of 1 M sorbitol. The cell suspension was mixed with 20 µL of β-mercaptoethanol and 20 mg of Zymolyase 100T (Seikagaku Kogyo Co., Japan), and incubated for 15 minutes at room temperature to degrade and remove the cell wall, thereby obtaining spheroplasts. Then, the spheroplasts were washed twice with 20 mL of 1 M sorbitol, and re-suspended in 20 mL of 1 M sorbitol. The re-suspended spheroplasts were mixed with 240 µL of 38% formaldehyde (Wako Chemicals, Osaka, Japan) and incubated at room temperature for 5 minutes to fix the spheroplasts. The fixed spheroplasts were washed twice with 20 mL of 1 M sorbitol and centrifuged at 5000 × g for 5 minutes to give a pellet. Then, the spheroplasts were re-suspended in 10 mL of nuclei preparation solution (10 mM HEPES-KOH, pH 7.5, 10 mM NaCl, 3 mM MgCl₂, and 0.5% [v/v] Nonidet P 40 substitute), and incubated at room temperature for 5 minutes. The nuclei were collected by centrifugation at 5000 × g for 5 minutes, and washed twice with 10 mL of nuclei wash solution (10 mM HEPES-KOH, pH 7.5, 10 mM NaCl, 3 mM MgCl₂). The nuclei were re-suspended in 10 mL of nuclei wash solution, and the suspension was dispensed in 500 µL volumes into 20 tubes, which were stored at -80°C until use.

The frozen nuclei suspension was thawed and re-suspended in 500 µL of 1× methylation buffer (50 mM Tris-HCl, pH 8.5, 50 mM NaCl, 1 mM DTT, 1.6 mM SAM). Then, the purified N-terminal 15 amino acid deletion mutant of M.CviQIX (referred to as CCMT; see the section "Results" described later) was added in an appropriate amount. After incubation at 37°C for 1 hour, the nuclei were centrifuged at 5000 × g for 1 minute to give a pellet, which was then re-suspended in 40 µL of 10 mM Tris-HCl, pH 8.0. This nuclei suspension was mixed with 5 µL of 20 mg/ml proteinase K and 10% (w/v) SDS, and incubated at 50°C for 1 hour. Then, this reaction was supplemented with 150 µL of 1 M Tris-HCl, pH 8.0, overlaid with a white mineral oil, and incubated at 80°C for 24 hours. The aqueous phase was transferred to a new test tube, and mixed with 200 µL of Buffer AL and isopropanol. This mixture was loaded onto a DNeasy column by centrifugation at 10,000 × g for 1 minute, and the column was washed sequentially with Buffer W1 and Buffer W2 (500 µL each). Finally, the purified DNA was eluted with 200 µL of Buffer AE, and provided for WGBS analysis using the tPBAT protocol. Buffer AL, Buffer W1, Buffer W2 and Buffer AE are all reagents included in a DNeasy Blood & Tissue Kit.

### NOMe-Seq of mammalian cells

IMR-90 cells (JCRB cell bank #JCRB9054) were cultured at 37°C in an atmosphere of 100% humidity and 5% CO₂ in four T75 flasks each containing 30 mL of minimum essential medium (Thermo Fisher Scientific) supplemented with 10% fetal bovine serum (Thermo Fisher Scientific) and 50 U/mL penicillin-streptomycin (Thermo Fisher Scientific). The cells grown were treated with trypsin and collected by centrifugation at 2000 × g for 5 minutes. The cells were washed twice with 20 mL of D-PBS (Nacalai Tesque, Inc., Japan), and re-suspended at 1 × 10⁷ cells/ml in CELLBANKER 1 (Takara Bio Inc., Japan). The cell suspension was dispensed in 500 µL volumes and stored at -80°C until use.

The frozen cell suspension was thawed before use and washed twice with PBS. Then, the cells were re-suspended in nuclei preparation buffer (10 mM Tris-HCl, pH 8.0, 10 mM NaCl, 3 mM MgCl₂, 0.1 mM EDTA) and 0.5% (w/v) NP-40, and incubated on ice for 10 minutes. Then, the nuclei were collected through centrifugation at 2000 × g for 5 minutes, re-suspended in 500 µL of 1× methylation buffer supplemented with an optimum amount of CCMT, and incubated at 37°C for 30 minutes. Then, the nuclei were collected by centrifugation at 2000 × g for 5 minutes, and the supernatant was removed. The nuclei were dissolved in 20 µL of 20 mg/ml proteinase K, and 200 µL of Buffer ATL was added thereto. After the reaction solution was incubated at 56°C for 1 hour, 200 µL of Buffer AL and 200 µL of isopropanol were added thereto. This mixture was loaded onto a DNeasy column, and washed with 500 µL of Buffer W1 or Buffer W2. The purified DNA was eluted with 200 µL of Buffer AE, and provided for WGBS analysis.

### 2. Results

### N-terminal deletion enhances the MTase activity of M.CviQIX and M.CviPII

The inventor of the present invention attempted to produce an active protein of M.CviQIX which has been reported on databases to have MTase activity at the first C of the CCD trinucleotide. However, DNA methylation activity was not detectable in most bacterial cells carrying this gene subcloned into several expression vectors (Figures 3A to 3C), but relatively strong MTase activity was observed in cells expressing M.CviQIX subcloned into expression vectors pCTF and pCNS. Namely, the genomic DNAs of cells carrying these two expression vectors showed resistance to both HaeIII and HpaII digestion (Figure 3C and Figures 7A and 7B). pCTF and pCNS are designed to produce a protein fused with a trigger factor (TF) and a SUMO motif at the N-terminal end of M.CviQIX, respectively (Figure 3A). TF and SUMO are expected to have an effect on protein solubilization and folding (Reference 22). Moreover, the genomic DNA extracted from the bacterial cells carrying the expression vector for N-terminal SUMO tagging showed stronger resistance to methylation-sensitive restriction enzymes than the genomic DNA extracted from bacterial cells carrying the expression vector for C-terminal SUMO tagging (Figure 3C). Thus, the inventor of the present invention attempted to purify the products of these N-terminal TF- and SUMO-tagged constructs. However, both proteins showed only weak MTase activity after affinity purification using the N-terminal His6 tag. These results suggested the possibility that some factor different from protein solubilization and folding would lie at the N-terminal end and control the DNA methylation activity of M.CviQIX. Thus, the inventor of the present invention hypothesized that the N-terminal sequence may cause the low methylation activity of purified M.CviQIX. For this purpose, analysis on the genomic DNAs of bacterial cells carrying expression vectors for N-terminal deletion mutants of M.CviQIX was performed. M.CviQIX has 3 methionine residues at the N-terminal end. Thus, the inventor of the present invention considered that any of these 3 residues would be used for translation initiation within the cells of organisms originally carrying this gene. For this purpose, 3 mutants were prepared to lack amino acid residues upstream of each methionine residue, and the methylation status of their host genome was analyzed. As a result, the deletion of N-terminal 11, 13 or 15 amino acids of M.CviQIX was found to enhance the methylation of the host genome (Figures 3D and 3E, and Figure 7C). Namely, each of these 3 methionine residues could be used as an initiation codon to produce active M.CviQIX. Likewise, similar results were also obtained in M.CviPII (Figures 7F and 7G). Namely, the N-terminal amino acid sequences of M.CviQIX and M.CviPII were found to have an inhibitory effect on DNA methylation activity.

### WGBS-based determination of recognition sequences in M.CviQIX and M.CviPII

The inventor of the present invention induced the expression of N-terminal 15 amino acid deletion (NDEL15) mutants of M.CviQIX and M.CviPII in *E. coli* cells, extracted the bacterial genomic DNAs, and performed whole genome bisulfite sequencing (WGBS) (Figure 4A). Methylation levels were measured for each cytosine residue, and the appearance frequency of surrounding nucleotide sequences contributing to the methylation levels were expressed logographically, thus indicating that M.CviQIX and M.CviPII introduced methylation at the first C of CC dinucleotide.

### Purification of MTases and confirmation of their activity in vitro

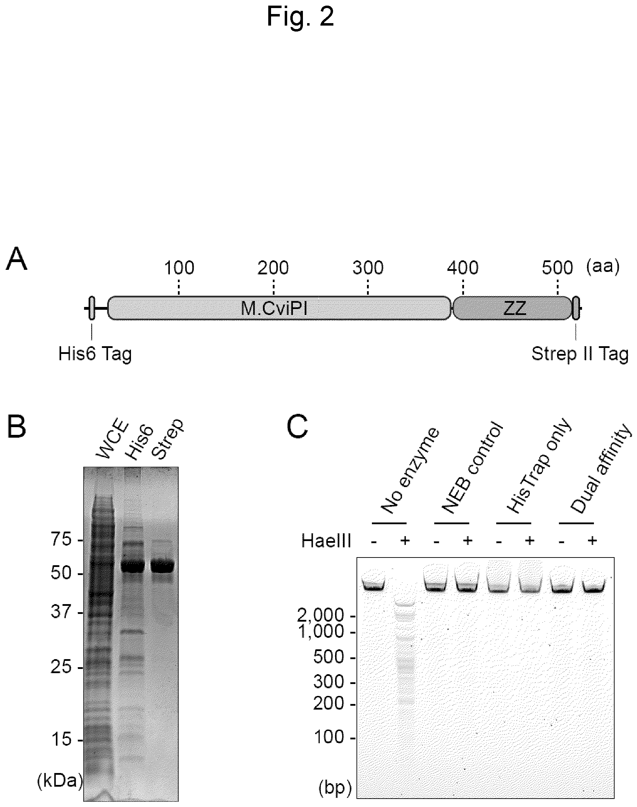

Subsequently, the inventor of the present invention attempted to purify active proteins by using genes for N-terminal 15 amino acid deletion mutants of M.CviQIX and M.CviPII. In this case, the inventor of the present invention also attempted to purify an active protein of M.CviPI, as a control, whose purification conditions had been established. Bacterial cells carrying an expression vector where each gene was cloned were cultured to induce protein expression, followed by dual affinity purification employing His- and StrepII-tags to purify these MTases (Figure 2). The proteins were stored in the presence of 100 µg/ml BSA, 10 mM DTT and 50% glycerol to preserve enzymatic activity during storage at -20°C. The purified proteins appeared highly homogenous upon SDS-PAGE. When assayed with restriction enzymes and WGBS using lambda phage DNA as a substrate, these MTases were found to cause the inhibition of DNA cleavage and thus confirmed to have methylation activity (Figure 8B).

### NOMe-Seq with a CC-recognizing MTase produces similar methylation patterns to GCMT

CC dinucleotide is a short recognition sequence which enables fine epigenome mapping at about 10 bp resolution. Thus, the inventor of the present invention attempted to establish a CC dinucleotide-recognizing MTase, M.CviQIX or M.CviPII, as a probe for epigenome mapping. Upon comparison of different combinations between expression vector and gene, M.CviQIX expressed under the control of a cold-shock promoter showed the highest yield and activity, and this protein was designated as CCMT. The salt concentration in CCMT-catalyzed methylation reaction was examined, thus finding that 50 mM sodium chloride was the best condition for stringent DNA methylation activity (Figure 9).

Then, CCMT was used for NOMe-Seq analysis of formaldehyde-crosslinked budding yeast nuclei. Formalin-fixed yeast spheroplasts were permeabilized in a buffer containing NP-40 to remove their cell membrane, and then subjected to CCMT-catalyzed methylation in the presence of S-adenosylmethionine, thereby achieving the methylation of DNA on chromatin. After genomic DNA was purified, the methylation status of this DNA was analyzed by WGBS.

Methylation levels in 10-bp bins were calculated and the methylation status of DNA between nuclei treated with commercially available GC dinucleotide-recognizing M.CviPI (GCMT) was compared with that of nuclei treated with CCMT, thus indicating that these two data sets showed similar distribution patterns of 5mC on the genome. It should be noted that since budding yeast has no endogenous DNA methylation, it can be interpreted that the detected D5mCs were all introduced by GCMT or CCMT. As illustrated in Figures 5A and 5B, a similar tendency was observed in genomic DNA methylation patterns. The aggregation plots of CCMT and GCMT signals were centered at several similar genomic features (Figures 5C and 5D, and Figure 10A). Sharp DNA methylation peaks were observed in the promoter regions of the genes (Figure 5B). These strong DNA methylation signals were co-localized well with the signals detected using ATAC-Seq. Thus, these signals were considered to indicate open chromatin regions. The peaks detected upon CCMT- and GCMT-based NOMe-Seq and ATAC-Seq were co-localized well (Figure 10B).

The DNA methylation levels at CC and GC dinucleotides aggregated well at the promoter-proximal regions (Figures 5C and 5D). These signals undoubtedly indicated nucleosome-free regions which are frequently observed in gene promoters as regions with no or weak nucleosome binding. The promoter-proximal DNA methylation signal at the -100 to -250 region of the transcription start site was inversely correlated with the low signal region detected through MNase-Seq (Figure 5E). The DNA methylation signals at the transcription start sites illustrated a periodicity of 160 to 170 bp, and were inversely correlated with those observed using MNase-Seq (Figures 5B and 5C to 5E). These observations recapitulated the previously reported properties of NOMe-Seq and MNase-seq (Reference 23).

### CCMT-based NOMe-Seq achieves fine mapping of the mammalian epigenomes

Subsequently, CCMT-based NOMe-Seq was applied to a human-derived culture cell line, IMR-90 cells. After the cells were treated with a buffer containing NP-40 to permeabilize their cell membrane, CCMT treatment was performed and genomic DNA was purified, which was then subjected to WGBS analysis. In addition, a sample not treated with CCMT was also provided and used as a control. High-quality methylome data with 24.5-fold and 25.7-fold coverage were obtained for CCMT-treated and untreated samples, respectively (Table 1).

**[Table 1]**

| **Basic statistics of methylome data in NOMe-Seq analysis on IMR90 cells** | | | | | |
|---|---|---|---|---|---|
| | | | Control | CCMT-treated | GCMT-treated |
| Accession numbers at NCBI GEO | | | GSM5769478 | GSM5769479 | GSM6111856 |
| Mapping | Read 1 | Number of reads | 462,135,990 | 445,678,391 | 447,579,538 |
| | | Unique map | 73.00% | 72.80% | 69.5% |
| | | Unmap | 24.20% | 24.70% | 27.9% |
| | Read 2 | Number of reads | 462,135,988 | 445,678,391 | 447,579,537 |
| | | Unique map | 65.10% | 64.90% | 63.6% |
| | | Unmap | 32.60% | 32.90% | 34.0% |
| Mapped read coverage | | Top | 12.9 | 12.3 | 11.4 |
| | | Bottom | 12.8 | 12.2 | 11.3 |
| | | Both | 25.7 | 24.5 | 22.7 |
| | | N | 12.9 | 12.3 | 11.3 |
| | | C | 14.2 | 13.4 | 12.4 |
| | | CpG | 14.2 | 13.1 | 12.0 |
| | | CHG | 14.4 | 13.6 | 12.8 |
| | | CHH | 14.1 | 13.4 | 12.5 |
| Methylation level | | C | 3.4 | 3.9 | 5.4 |
| | | CpG | 56.2 | 56.2 | 58.0 |
| | | CHG | 1 | 1.2 | 3.1 |
| | | CHH | 0.8 | 1.5 | 2.8 |
| Coverage vs read depth | | >=1 | 86.2% | 86.1% | 85.7% |
| | | >=3 | 84.0% | 83.8% | 82.2% |
| | | >=5 | 80.9% | 80.6% | 76.9% |
| | | >=10 | 64.8% | 63.2% | 55.5% |

As expected, intrinsic DNA methylation levels at CG sites were almost identical between CCMT-treated cells and untreated cells (Figures 6A and 6B). In contrast, DNA methylation levels at CC sites increased only in the CCMT-treated nuclei (Figures 6C and 6D). These results indicated that endogenous CG methylation and CCMT-induced methylation at CC dinucleotides could be explicitly separated from each other.

Then, the two data sets were visually compared using a genome browser. As illustrated in Figure 6E, several large domains with different intrinsic DNA methylation levels were observed in both data sets. These domains were undoubtedly highly methylated regions and partially methylated domains, and their existence has been described for IMR-90 cells (Reference 24). The comparison between CCMT-based NOMe-Seq and untreated control indicated that these two data sets were observed in almost the same status of intrinsic methylation on CG dinucleotides in mammalian cells. In contrast, strong and sharp methylation peaks were observed for CC methylation in the CCMT-treated samples, whereas no peaks were observed in the untreated control (Figure 6E). Moreover, these peaks largely co-localized with the peaks identified using ATAC-Seq. Thus, the peaks observed in the CCMT-induced methylation signals indicated highly accessible chromatin regions (Figure 6E). These results strongly suggested that CCMT-based NOMe-Seq would achieve simultaneous measurement of both the intrinsic methylome and chromatin accessibility of mammalian cells.

### 3. Discussion

Despite the increasing demand for MTases as tools for epigenome mapping, the available enzymes are limited particularly for cytosine C-5 MTases of short recognition sequences. This example elucidated that the methylation activity of M.CviQIX and M. CviPII was inhibited by their N-terminal amino acid sequences of several residues, and conversely indicated that their N-terminal deletion mutants had high DNA methylation activity. These artificial mutant proteins could be purified and were found to retain their activity even *in vitro.* Moreover, they were also found to target CC dinucleotide which is a novel recognition sequence for cytosine C-5 MTases.

This CC dinucleotide never overlaps with CG dinucleotide which is a recognition sequence for mammalian endogenous MTases. Thus, in addition to mammalian endogenous MTases, such a mutant protein was deemed to have a great advantage as a probe in epigenetic mapping for simultaneous detection of any epigenomic states. This was demonstrated by the achievement of NOMe-Seq in this example.

The individual references cited herein are listed below.
1. Greenberg, M.V.C. and Bourc'his, D. (2019) The diverse roles of DNA methylation in mammalian development and disease. Nature Reviews Molecular Cell Biology, 20, 590-607.
2. Edwards, J.R., Yarychkivska, O., Boulard, M. and Bestor, T.H. (2017) DNA methylation and DNA methyltransferases. Epigenetics & Chromatin, 10, 23.
3. Jones, P.A. (2012) Functions of DNA methylation: islands, start sites, gene bodies and beyond. Nature Reviews Genetics, 13, 484-492.
4. Petryk, N., Bultmann, S., Bartke, T. and Defossez, P.-A. (2021) Staying true to yourself: mechanisms of DNA methylation maintenance in mammals. Nucleic Acids Res., 49, 3020-3032.
5. Jones, P.A. and Liang, G. (2009) Rethinking how DNA methylation patterns are maintained. Nat Rev Genet, 10, 805-811.
6. Edwards, J.R., Yarychkivska, O., Boulard, M. and Bestor, T.H. (2017) DNA methylation and DNA methyltransferases. Epigenetics Chromatin, 10, 23.
7. Tajima, S., Suetake, I., Takeshita, K., Nakagawa, A. and Kimura, H. (2016) Domain Structure of the Dnmt1, Dnmt3a, and Dnmt3b DNA Methyltransferases. Adv. Exp. Med. Biol., 945, 63-86.
8. Jang, H.S., Shin, W.J., Lee, J.E. and Do, J.T. (2017) CpG and Non-CpG Methylation in Epigenetic Gene Regulation and Brain Function. Genes, 8, 148.
9. Patil, V., Ward, R.L. and Hesson, L.B. (2014) The evidence for functional non-CpG methylation in mammalian cells. Epigenetics, 9, 823-828.
10. van de Lagemaat, L.N., Flenley, M., Lynch, M.D., Garrick, D., Tomlinson, S.R., Kranc, K.R. and Vernimmen, D. (2018) CpG binding protein (CFP1) occupies open chromatin regions of active genes, including enhancers and non-CpG islands. Epigenetics & Chromatin, 11, 59.
11. Teissandier, A. and Bourc'his, D. (2017) Gene body DNA methylation conspires with H3K36me3 to preclude aberrant transcription. The EMBO Journal, 36, 1471-1473.
12. Dhayalan, A., Rajavelu, A., Rathert, P., Tamas, R., Jurkowska, R.Z., Ragozin, S. and Jeltsch, A. (2010) The Dnmt3a PWWP domain reads histone 3 lysine 36 trimethylation and guides DNA methylation. The Journal of biological chemistry, 285, 26114-26120.
13. Solomon, M.J., Larsen, P.L. and Varshavsky, A. (1988) Mapping protein-DNA interactions in vivo with formaldehyde: evidence that histone H4 is retained on a highly transcribed gene. Cell, 53, 937-947.
14. Johnson, D.S., Mortazavi, A., Myers, R.M. and Wold, B. (2007) Genome-wide mapping of in vivo protein-DNA interactions. Science, 316, 1497-1502.
15. Kelly, T.K., Liu, Y., Lay, F.D., Liang, G., Berman, B.P. and Jones, P.A. (2012) Genome-wide mapping of nucleosome positioning and DNA methylation within individual DNA molecules. Genome Res., 22, 2497-2506.
16. Lee, I., Razaghi, R., Gilpatrick, T., Molnar, M., Gershman, A., Sadowski, N., Sedlazeck, F.J., Hansen, K.D., Simpson, J.T. and Timp, W. (2020) Simultaneous profiling of chromatin accessibility and methylation on human cell lines with nanopore sequencing. Nat Methods, 17, 1191-1199.
17. Stergachis, A.B., Debo, B.M., Haugen, E., Churchman, L.S. and Stamatoyannopoulos, J.A. (2020) Single-molecule regulatory architectures captured by chromatin fiber sequencing. Science, 368, 1449-1454.
18. Steensel, B.v. and Henikoff, S. (2000) Identification of in vivo DNA targets of chromatin proteins using tethered Dam methyltransferase. Nat. Biotechnol., 18, 424-428.
19. Roberts, R.J. and Macelis, D. (1997) REBASE-restriction enzymes and methylases. Nucleic Acids Res., 25, 248-262.
20. Roberts, R.J., Vincze, T., Posfai, J. and Macelis, D. (2015) REBASE--a database for DNA restriction and modification: enzymes, genes and genomes. Nucleic Acids Res., 43, D298-D299.
21. Xu, M., Kladde, M.P., Van Etten, J.L. and Simpson, R.T. (1998) Cloning, characterization and expression of the gene coding for a cytosine-5-DNA methyltransferase recognizing GpC. Nucleic Acids Res., 26, 3961-3966.
22. Malakhov, M.P., Mattern, M.R., Malakhova, O.A., Drinker, M., Weeks, S.D. and Butt, T.R. (2004) SUMO fusions and SUMO-specific protease for efficient expression and purification of proteins. Journal of Structural and Functional Genomics, 5, 75-86.
23. Klemm, S.L., Shipony, Z. and Greenleaf, W.J. (2019) Chromatin accessibility and the regulatory epigenome. Nature Reviews Genetics, 20, 207-220.
24. Lister, R., Pelizzola, M., Dowen, R.H., Hawkins, R.D., Hon, G., Tonti-Filippini, J., Nery, J.R., Lee, L., Ye, Z., Ngo, Q.M. et al. (2009) Human DNA methylomes at base resolution show widespread epigenomic differences. Nature, 462, 315-322.
25. Braberg, H., Echeverria, I., Bohn, S., Cimermancic, P., Shiver, A., Alexander, R., Xu, J., Shales, M., Dronamraju, R., Jiang, S. et al. (2020) Genetic interaction mapping informs integrative structure determination of protein complexes. Science, 370.
26. Miura, F., Fujino, T., Kogashi, K., Shibata, Y, Miura, M., Isobe, H. and Ito, T. (2018) Triazole linking for preparation of a next-generation sequencing library from single-stranded DNA. Nucleic Acids Res., 46, e95.

### Industrial Applicability

The present invention enables the provision of a protein (MTase) that is capable of introducing methylation which is distinguishable from intrinsic DNA methylation (endogenous methylation) in mammalian cells, more particularly capable of specifically recognizing a "CC dinucleotide sequence" and 5-methylating the 5'-position cytosine residue in the sequence, as well as a gene encoding the same, etc. The protein (MTase) according to the present invention is useful in terms of theoretically achieving, e.g., a method for epigenome analysis (epigenome mapping) which allows simultaneous profiling of endogenous methylation and any other epigenomes.

### Sequence Listing Free Text

SEQ ID NO: 5: recombinant DNA
SEQ ID NO: 6: recombinant protein
SEQ ID NO: 7: recombinant DNA
SEQ ID NO: 8: recombinant protein
SEQ ID NO: 9: synthetic DNA
SEQ ID NO: 10: synthetic DNA

## Claims

1. A protein as set forth in (a), (b) or (c) below:
(a) a protein modified to delete some amino acid residues including the N-terminal end of DNA methyltransferase M.CviQIX or M.CviPII and thereby acquire the ability to specifically recognize a CC dinucleotide sequence and 5-methylate the 5'-position cytosine residue in the sequence;
(b) a protein comprising an amino acid sequence with deletion, substitution or addition of one or several amino acids in the amino acid sequence of the protein (a) and having the ability to specifically recognize a CC dinucleotide sequence and 5-methylate the 5'-position cytosine residue in the sequence; or
(c) a protein comprising an amino acid sequence sharing an identity of 80% or more with the amino acid sequence of the protein (a) and having the ability to specifically recognize a CC dinucleotide sequence and 5-methylate the 5'-position cytosine residue in the sequence.

2. A protein as set forth in (a), (b) or (c) below:
(a) a protein having the amino acid sequence as set forth in SEQ ID NO: 6 or 8;
(b) a protein comprising an amino acid sequence with deletion, substitution or addition of one or several amino acids in the amino acid sequence of the protein (a) and having the ability to specifically recognize a CC dinucleotide sequence and 5-methylate the 5'-position cytosine residue in the sequence; or
(c) a protein comprising an amino acid sequence sharing an identity of 80% or more with the amino acid sequence of the protein (a) and having the ability to specifically recognize a CC dinucleotide sequence and 5-methylate the 5'-position cytosine residue in the sequence.

3. A gene encoding the protein according to claim 1 or 2.

4. A gene comprising DNA as set forth in (a), (b) or (c) below:
(a) DNA comprising the nucleotide sequence as set forth in SEQ ID NO: 5 or 7;
(b) DNA which is hybridizable under stringent conditions with DNA consisting of a nucleotide sequence complementary to the DNA (a) and which encodes a protein having the ability to specifically recognize a CC dinucleotide sequence and 5-methylate the 5'-position cytosine residue in the sequence; or
(c) DNA which shares an identity of 80% or more with the DNA (a) and which encodes a protein having the ability to specifically recognize a CC dinucleotide sequence and 5-methylate the 5'-position cytosine residue in the sequence.

5. A recombinant vector comprising the gene according to claim 3 or 4.

6. A transformant comprising the recombinant vector according to claim 5.

7. A process for producing a protein having the ability to specifically recognize a CC dinucleotide sequence and 5-methylate the 5'-position cytosine residue in the sequence, which comprises the steps of:
culturing the transformant according to claim 6; and
collecting the protein from the resulting cultured material.

8. A method for epigenome analysis, which comprises using the protein according to claim 1 or 2 or a protein produced by the process according to claim 7.
